# EUROPEAN PATENT APPLICATION

(11) **EP 4 353 220 A1**
(43) Date of publication of application: **17.04.2024**
(21) Application number: 22306548.3
(22) Date of filing: 12.10.2022
(51) Int. Cl.: A61K 9/00, A61K 47/18, A61K 47/26, A61K 47/50

(54) **USE OF A LIQUID AQUEOUS COMPOSITION FOR SOLUBILIZATION AND STABILIZATION OF AN ANTIBODY-DRUG CONJUGATE**

(71) Applicant: PIERRE FABRE MEDICAMENT, 81500 Lavaur (FR)
(72) Inventor: BECK, Alain, 81106 CASTRES (FR)
(74) Representative: Regimbeau

(57) **Abstract**

The present invention relates to the use of a liquid aqueous composition comprising a tonicity agent, a buffering agent, a non-ionic surfactant and a polysaccharide, and having a pH of from 5 to 7, for solubilization and stabilization of an antibody-drug conjugate during a period T equal or greater than 3 months at a temperature Θ of from 2°C to 8°C.

## Description

### TECHNICAL FIELD

The present invention relates to the use of a liquid aqueous composition for solubilization and stabilization of an antibody-drug conjugate (ADC) during at least 3 months at a temperature of from 2°C to 8°C.

### BACKGROUND

Many antibody-drug conjugates (ADCs) existing on the market are used for treatment purposes, in particular cancer. Different ADCs are increasingly developed and used to treat different types of cancers. The administration of ADCs to reduce tumor growth represents a real future targeted therapy. ADCs comprising an antibody linked to a drug via a linker are manufactured and stored in a lyophilized form. Indeed, 11 ADCs approved by the FDA are offered on the market in lyophilized form in order to preserve the product for a long time without any degradation. Stability is an essential and critical parameter for this type of product. In other words, an ADC must be stable to maintain its physicochemical properties such as a stable DAR, avoidance of aggregates, stable pH, etc. before administration. The lyophilization is currently the only way to sustainably preserve this product while maintaining good stability.

Lyophilization enabling to preserve ADC is a known method of drying product. Indeed, the lyophilized form minimizes the instability associated with the linker which connects the drug and the antibody during storage. Before being lyophilized, the product must be frozen and then undergoes successive vacuum drying to become a lyophilizate that can be stored for a certain time. This method is therefore costly in terms of time but also energy.

Moreover, when ADC is to be administered to a patient, it is necessary to extemporaneously solubilize it. Solubilization step must be done by a practitioner and can generate error and loss of time.

Therefore, there is a real need to provide ADC which can be used simply and quickly while keeping it stable during several months, preferably at least 12 months. No existing ADC on the market enables the practitioner to use an ADC in a solubilized form in a simple way without any manipulation to be carried out before administration to a patient.

### SUMMARY OF THE INVENTION

Very surprisingly, an ADC has been found to be stable in solution for a period greater than 3 months and extremely surprisingly even up to 48 months, without any degradation of the ADC and without aggregate. The present invention has made it possible that an ADC can be stored for several months in the form of an aqueous liquid composition in a soluble form. The prior art suggests until today that ADCs are only stable when they are in a lyophilized form. However, surprisingly it appears that an ADC in a solution can be stable. The invention therefore goes against what is currently taught, namely that an ADC is only stable in the lyophilization form.

Thus, the invention has great advantages. The invention proposes a ready to use preparation comprising ADC to be administrated to a patient. The administration of ADC solution in a ready to use preparation is greatly facilitated for practitioners since it no longer requires a solubilization and thus avoid loss of time. The solubilization step before administration is deleting, thus enabling to reduce errors before administration. Moreover, the preparation may already be ready to use in a syringe to be injected into the patient or in a sterile pouch of NaCl 0.9% solution. This ready to use preparation improves the storage conditions and uses.

The present invention relates thus to the use of a liquid aqueous composition comprising a tonicity agent, a buffering agent, a non-ionic surfactant and a polysaccharide, and having a pH of from 5 to 7, for solubilization and stabilization of an antibody-drug conjugate during a period T equal or greater than 3 months at a temperature Θ of from 2°C to 8°C.

The present invention relates also to a method for solubilizing and stabilizing an antibody-drug conjugate during a period T equal or greater than 3 months at a temperature Θ of from 2°C to 8°C comprising:
solubilizing the antibody-drug conjugate in a liquid aqueous composition comprising a tonicity agent, a buffering agent, a non-ionic surfactant and a polysaccharide, and having a pH of from 5 to 7 in order to obtain a solution, and
storing the solution during a period T equal or greater than 3 months at a temperature Θ of from 2°C to 8°C.

### DETAILED DESCRIPTION

### Liquid aqueous composition

The liquid aqueous composition used in the present invention comprises a tonicity agent, a buffering agent, a non-ionic surfactant and a polysaccharide, and has a pH of from 5 to 7, preferably from 6 to 7 and most preferably of about 6.5. The pH can be adjusted by addition of an acid such as HCI (hydrochloric acid).

The term "about" means in the context of the present invention that the concerned value may be lower or higher by 10%, especially by 5%, in particular by 1%, than the value indicated.

The term "tonicity agent" also named "tonicity adjuster" is intended to mean an agent which, when present in a solution, is capable to modify the tonicity of the solution, i.e. its capability to modify the volume of cells by altering their water content. The tonicity agent can be a ionic or non-ionic tonicity agent. The tonicity agent is used in the liquid aqueous composition according to the invention in order to ensure that said composition comprising also the antibody-drug conjugate according to the invention is isotonic with blood serum. Indeed, such an isotonic solution has the same osmotic pressure as blood serum so that it does not affect the membranes of the red blood cells, and thus avoids patient discomfort, biological damage to the patient or ineffective treatment by preventing osmotic shock at the site of application.

According to a particular embodiment, the tonicity agent is selected in the group consisting of polyols, inorganic salts, non aromatic amino acids, and mixtures thereof.

The term "polyol" as used in the present invention refers to an organic compound comprising at least two hydroxyl (OH) groups. It refers more particularly to an alkane comprising x1 carbon atoms and substituted with x2 hydroxyl groups, with x1 an integer from 2 to 10, notably from 3 to 6 and x2 an integer from 2 to x1, preferably equal to x1. The polyol may respond to the formula CₙH₂ₙ₊₂Oₙ with n = x1 = x2. The polyol can be selected in the group consisting of glycerol, erythritol, arabitol, xylitol, sorbitol, mannitol, and mixtures thereof.

The inorganic salt can be an alkali metal (e.g. Na or K) sulfate, an alkali metal (e.g. Na or K) halide such as chloride, an alkaline earth metal (e.g. Ca or Mg) sulfate, an alkaline earth metal (e.g. Ca or Mg) halide such as chloride, or a mixture thereof. In particular, the inorganic salt is selected in the group consisting of sodium chloride, sodium sulphate, potassium chloride, potassium sulphate, magnesium chloride, magnesium sulphate, calcium chloride, calcium sulphate, and mixtures thereof. Preferably, the inorganic salt is NaCl, MgCl₂ or CaCl₂. Most preferably, the inorganic salt is NaCl.

The term "non aromatic amino acid" as used in the present invention refers to amino acids which do no comprise an aromatic ring, and in particular natural non aromatic α-amino acids (e.g. Alanine (Ala), Arginine (Arg), Asparagine (Asn), Aspartic acid (Asp), Cysteine (Cys), Glutamine (Gln), Glutamic acid (Glu), Glycine (Gly), Isoleucine (Ile), Leucine (Leu), Lysine (Lys), Méthionine (Met), Proline (Pro), Serine (Ser), Threonine (Thr), and Valine (Val)) in the D or L form, as well as non-natural non aromatic amino acids (e.g. β-alanine, allylglycine, tert-leucine, 3-amino-adipic acid, 2-aminobutanoic acid, 4-amino-1-carboxymethyl piperidine, 1-amino-1-cyclobutanecarboxylic acid, 4-aminocyclohexaneacetic acid, 1-amino-1-cyclohexanecarboxyilic acid, (1R,2R)-2-aminocyclohexanecarboxylic acid, (1R,2S)-2-aminocyclohexanecarboxylic acid, (1S,2R)-2-aminocyclohexanecarboxylic acid, (1S,2S)-2-aminocyclohexanecarboxylic acid, 3-aminocyclohexanecarboxylic acid, 4-aminocyclohexanecarboxylic acid, (1R,2R)-2-aminocyclopentanecarboxylic acid, (IR,2S)-2-aminocyclopentanecarboxyilic acid, 1-amino-1-cyclopentanecarboxylic acid, 1-amino-1-cyclopropanecarboxylic acid, 2-aminobutanoic acid, 4-aminobutanoic acid, 6-aminohexanoic acid, (4R,5S)-4-amino-5-methylheptanoic acid, (R)-4-amino-5-methylhexanoic acid, (R)-4-amino-6-methylthiohexanoic acid, (S)-4-amino-pentanoic acid, (R)-4-amino-5-phenylpentanoic acid, (4R,5R)-4-amino-5-hydroxyhexanoic acid, (R)-4-amino-5-hydroxypentanoic acid, 8-aminooctanoic acid, (2S,4R)-4-amino-pyrrolidine-2-carboxylic acid, (2S,4S)-4-amino-pyrrolidine-2-carboxylic acid, azetidine-2-carboxylic acid, (S)-4,8-diaminooctanoic acid, tert-butylglycine acid, γ-carboxyglutamate, β-cyclohexylalanine, citrulline, 2,3-diamino propionic acid, homocyclohexylalanine, 4-hydroxyproline, isonipecotic acid, α-methyl-alanine, nicopetic acid, norleucine, norvaline, octahydroindole-2-carboxylic acid, ornithine, penicillamine, pipecolic acid, propargylglycine, sarcosine, or tranexamic acid). Preferably, it will be a natural or non-natural non aromatic α-amino acid and preferably a natural non aromatic α-amino acid.

According to a particular embodiment, the tonicity agent is a polyol selected in the group consisting of glycerol, erythritol, arabitol, xylitol, sorbitol, mannitol, and mixtures thereof.

According to another embodiment, the tonicity agent is an inorganic salt selected in the group consisting of an alkali metal (e.g. Na or K) sulfate, an alkali metal (e.g. Na or K) halide such as chloride, an alkaline earth metal (e.g. Ca or Mg) sulfate, an alkaline earth metal (e.g. Ca or Mg) halide such as chloride, and mixtures thereof; in particular selected in the group consisting of sodium chloride, sodium sulphate, potassium chloride, potassium sulphate, magnesium chloride, magnesium sulphate, calcium chloride, calcium sulphate, and mixtures thereof; preferably being NaCl, MgCl₂ or CaCl₂; most preferably being NaCl. The inorganic salt, especially when it is NaCl, can be used at a concentration of from 100 mM to 200 mM, preferably at about 150 mM (concentration in the liquid aqueous composition).

The buffering agent is intended to maintain the pH of the composition in the range from 5 to 7, preferably from 6 to 7 and most preferably at about 6.5.

According to a particular embodiment, the buffering agent is selected in the group consisting of citrate buffer, phosphate buffer, and histidine buffer, and preferably is a histidine buffer. The buffering agent, especially when it is a histidine buffer, can be used at a concentration of from 5 mM to 50 mM, preferably at about 25 mM (concentration in the liquid aqueous composition).

The non-ionic surfactant can be a polysorbate which is an ethoxylated sorbitan esterified with fatty acids. The polysorbate can be selected in the group consisting of Polysorbate 20 and Polysorbate 80, and preferably is Polysorbate 80. The non-ionic surfactant, especially when it is a polysorbate such as Polysorbate 80, can be used at a concentration of from 0.0001 to 0.01% (v/v), preferably at about 0,005% (v/v), based on the volume of the liquid aqueous composition.

The term "polysaccharide" as used in the present invention refers to a chain comprising at least 2 saccharides bound together by means of OH functions. According to a particular embodiment, the polysaccharide is a disaccharide, and preferably is sucrose. The polysaccharide, especially when it is a disaccharide such as sucrose, can be used at a concentration of from 0.1% to 10% (w/v), preferably at about 6% (w/v), based on the volume of the liquid aqueous composition.

According to a particular embodiment, the liquid aqueous composition comprises NaCl, a histidine buffer, Polysorbate 80, and sucrose; especially comprises 150 mM NaCl, 25 mM Histidine buffer, 0.005% Polysorbate 80 (v/v), and 6% sucrose (w/v); and preferably has a pH of about 6.5.

### Antibody-drug conjugate (ADC)

The present disclosure provides an antibody-drug conjugate (interchangeably referred to as "immunoconjugate," or "ADC") comprising an antibody or an antigen binding fragment thereof as described herein, said antibody being conjugated to a drug, and especially to a cytotoxic agent.

Many cytotoxic agents have been isolated or synthesised and make it possible to inhibit the cells proliferation, or to destroy or reduce, if not definitively, at least significantly the tumour cells. However, the toxic activity of these agents is not limited to tumour cells, and the non-tumour cells are also affected and can be destroyed. More particularly, side effects are observed on rapidly renewing cells, such as haematopoietic cells or cells of the epithelium, in particular of the mucous membranes.

For more detail, the person skilled in the art may refer to the manual edited by the "Association Française des Enseignants de Chimie Thérapeutique" and entitled "Traité de chimie thérapeutique, vol. 6, Médicaments antitumoraux et perspectives dans le traitement des cancers", edition TEC & DOC, 2003.

The invention thus concerns an antibody drug conjugate (ADC) comprising an antibody or an antigen binding fragment thereof covalently linked to a drug, preferably by means of a linker. The ADC may have the following formula (I):

**Ab**-(**L**-**D**)**ₙ** (I)

or a pharmaceutically acceptable salt thereof,
wherein
**Ab** is an antibody, or an antigen binding fragment thereof,
**L** is a linker, and in particular a cleavable or non-cleavable linker;
**D** is a drug moiety,
and n is an integer from 1 to 12, in particular from 1 to 8, preferably from 2 to 6 and more preferably of 4 ± 0.5. n is the number of drug moieties **D** linked to **Ab.** It corresponds to the DAR (Drug-to-Antibody ratio).

According to a particular embodiment, the DAR is from 1 to 12, in particular from 1 to 8, preferably from 2 to 6 and more preferably of 4 ± 0.5.

In the present invention by "pharmaceutically acceptable" is meant that which can be used in the preparation of a pharmaceutical composition which is generally, safe nontoxic and neither biologically nor otherwise undesirable, and which is acceptable for veterinary use as well as for human pharmaceutical use.

By "pharmaceutically acceptable salt" of a compound is meant a salt which is pharmaceutically acceptable as defined herein and which has the desired pharmacological activity of the parent compound.

Pharmaceutically acceptable salts notably comprise:
(1) the addition salts of a pharmaceutically acceptable acid formed with pharmaceutically acceptable inorganic acids such as hydrochloric, hydrobromic, phosphoric, sulfuric and similar acids; or formed with pharmaceutically acceptable organic acids such as acetic, trifluoroacetic, propionic, succinic, fumaric, malic, tartaric, citric, ascorbic, maleic, glutamic, benzoic, salicylic, toluenesulfonic, methanesulfonic, stearic, lactic and similar acids; and
(2) the addition salts of a pharmaceutically acceptable base formed when an acid proton present in the parent compound is either replaced by a metallic ion e.g. an alkaline metal ion, an alkaline-earth metal ion or an aluminium ion; or coordinated with a pharmaceutically acceptable organic base such as lysine, arginine and similar; or with a pharmaceutically acceptable inorganic base such as sodium hydroxide, potash, calcium hydroxide and similar.

### Drug

The drug can be a small molecule, such as a peptide-based drug including for example a dolastatin, an auristatin (particularly the monomethylauristatine E or F (MMAE or MMAF)), an amatoxin (especially an amanitin) and their derivatives.

According to a first embodiment, the drug is an amatoxin, such as amanitin, or a derivative thereof. It can have the following formula (I): 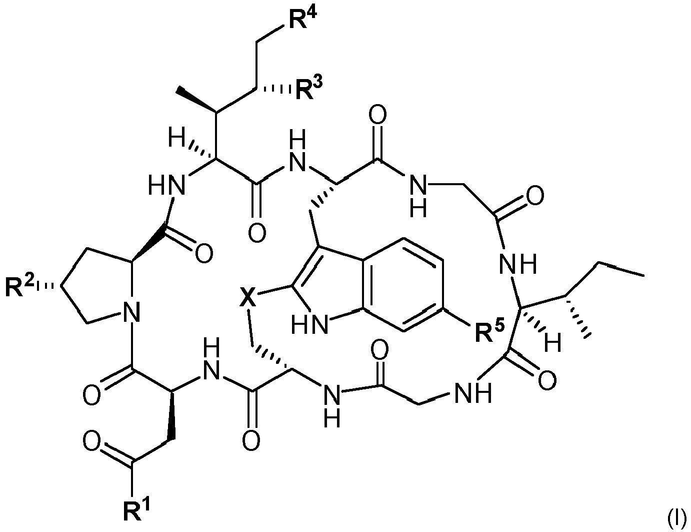 wherein:
R¹ is OH or NH₂,
R² is OH or H, preferably OH,
R³ is OH or H, preferably OH,
R⁴ is OH or H,
R⁵ is OH or H, and
X is S, SO or SO₂, preferably S or SO.

It can be an amanitin, such as α-amanitin, β-amanitin, γ-amanitin or ε-amanitin of the following formulas:

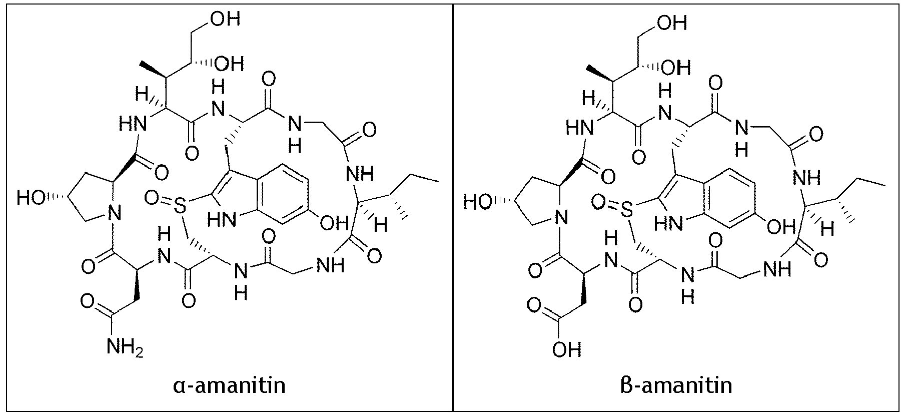

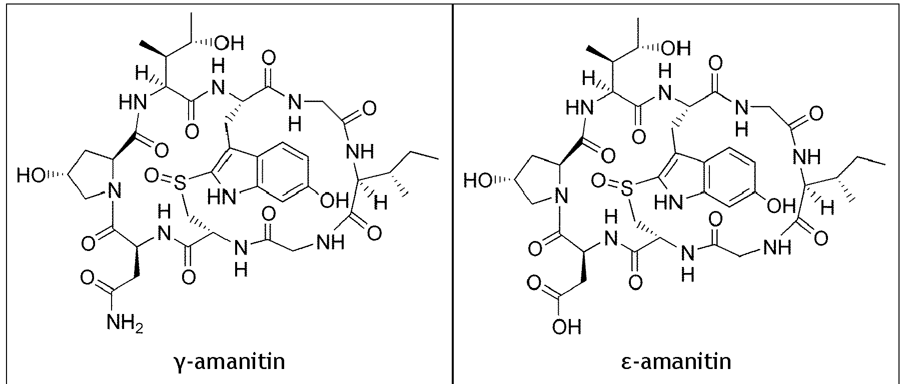

It can be also a derivative thereof (HDP 30.2105) of the following formula, which can be prepared as disclosed in WO2018/115466:

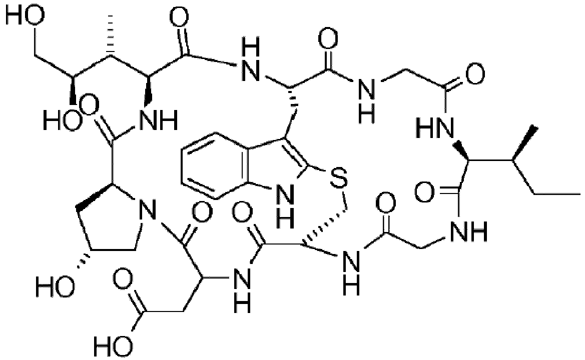

The amatoxin of the derivative thereof can be linked to the linker L by any OH or NH₂ group present on the drug such as by the R¹, R², R³, R⁴ or R⁵ group when it does not represent H, in particular by the R¹ or R⁵ group when it does not represent H, preferably by the R¹ group.

According to a second embodiment, the drug is an auristatin (e.g. MMAE or MMAF) or a derivative thereof. Thus, the drug moiety D may have the following formula (II): 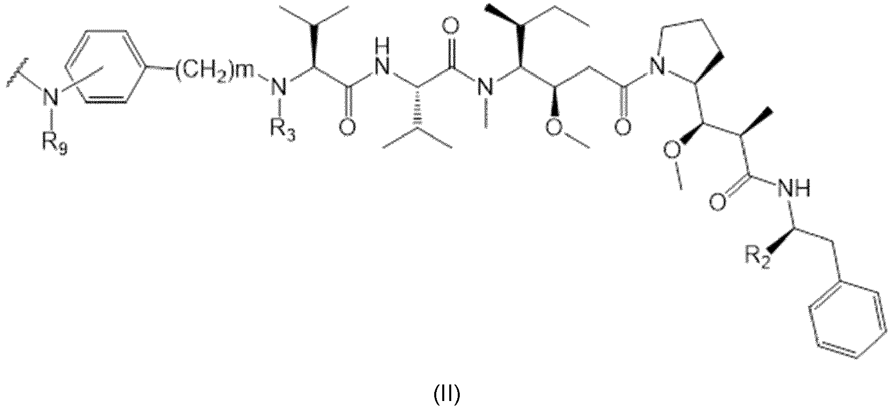 wherein:
R₂ is COOH, COOCH₃ or thiazolyl,
R₃ is H or (C₁-C₆)alkyl,
R₉ is H or (C₁-C₆)alkyl,
m is an integer comprised between 1 and 8 and
the wavy line indicates the point of attachment to L.

The drug moiety of formula (II) can be prepared as described in WO2015162291.

By "alkyl" in the present invention is meant a straight-chain or branched, saturated hydrocarbon chain. For example, mention can be made of methyl, ethyl, propyl, isopropyl, butyl, isobutyl, *sec*-butyl, *tert*-butyl*,* pentyl or hexyl groups.

By "(Cₓ-C_{y})alkyl" in the present invention is meant an alkyl chain such as defined above comprising x to y carbon atoms. Therefore, a (C₁-C₆)alkyl group is an alkyl chain having 1 to 6 carbon atoms.

The (C₁-C₆)alkyl is advantageously a (C₁-C₄)alkyl, preferably a (C₁-C₂)alkyl.

According to a particular embodiment, R₂ represents a COOH group.

According to another particular embodiment, R₂ is a thiazole (in particular a thiazol-2-yl group).

According to another particular embodiment, R₂ is COOMe.

According to one particular embodiment of the present invention, R₂ is more particularly a COOH, COOMe or thiazol-2-yl group.

R₃ particularly represents a (C₁-C₆)alkyl,advantageously a methyl group.

m is an integer comprised between 1 and 8, in particular between 1 and 6, advantageously between 1 and 4, preferably is 1 or 2.

In a particular embodiment, R₂ is COOH, R₃ is a methyl group and m is 1 or 2. Rg may be a methyl group or a hydrogen.

In a particular embodiment:
- R₂ is COOH, R₃ is a methyl group, Rg is a methyl group and m is 1 or 2, or
- R₂ is COOH, R₃ is a methyl group, R₉ is a hydrogen and m is 1 or 2.

The NR₉ group may be located on the phenyl ring in a para position in relation to the (CH₂)ₘ group.

The drug moiety D can be more particularly:

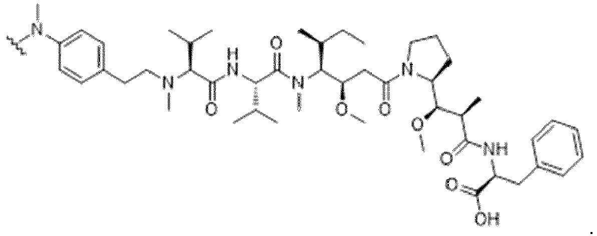

### Linker

The antibody drug conjugate described herein may comprise a linker.

"Linker", "Linker Unit", or "link" means a chemical moiety comprising a chain of atoms that covalently attaches an antibody or an antigen binding fragment thereof to a drug.

Linkers may be made using a variety of bifunctional protein coupling agents such as N-succinimidyl-3-(2-pyridyldithio)propionate (SPDP), succinimidyl-4-(N-maleimidomethyl)cyclohexane-1-carboxylate (SMCC), iminothiolane (IT), bifunctional derivatives of imidoesters (such as dimethyl adipimidate HCI), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Carbon-14-labelled 1-isothiocyanatobenzyl-3-methyldiethylene triaminepentaacetic acid (MX-DTPA) is an exemplary chelating agent for conjugation of cytotoxic agents to the addressing system. Other cross-linker reagents may be BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, III., U.S.A).

The linker may be a "non-cleavable" or "cleavable" linker. "The ideal linker should not induce ADC aggregation and can prevent premature release of payload in plasma and can be effectively released at desired targeted sites. "(https://www.biochempeg.com/article/87.html) Most ADC comprise a cleavable linker.

In an embodiment, the linker is a "non-cleavable linker" which reduces the off-target toxicity caused by the premature release of cytotoxic small molecule into the bloodstream. This linker must be sufficiently stable to avoid systemic toxicity. An ADC comprising a non-cleavable linker relies on the complete lysosomal proteolytic degradation of the antibody that releases the antibody-drug after internalization.

In a preferred embodiment, the linker is a "cleavable linker" facilitating release of the drug in the cell. For example, an acid-labile linker, peptidase-sensitive linker, photolabile linker, dimethyl linker or disulfide-containing linker may be used. The linker is, in a preferred embodiment, cleavable under intracellular conditions, such that cleavage of the linker releases the drug from the antibody in the intracellular environment.

For example, in some embodiments, the linker may be cleaved by a cleaving agent that is present in the intracellular environment (e.g., within a lysosome or endosome or caveolae). The linker can be, for example, a peptidyl linker that is cleaved by an intracellular peptidase or protease enzyme, including a lysosomal or endosomal protease. Typically, the peptidyl linker is at least two amino acids long or at least three amino acids long. Cleaving agents can include cathepsins B and D and plasmin, all of which are known to hydrolyse dipeptide drug derivatives resulting in the release of active drug inside target cells. For example, a peptidyl linker that is cleavable by the thiol-dependent protease cathepsin-B, which is highly expressed in cancerous tissue, can be used (e.g., a Phe-Leu or a Gly-Phe-Leu-Gly linker). In specific embodiments, the peptidyl linker cleavable by an intracellular protease is a Val-Cit linker or a Phe-Lys linker. One advantage of using intracellular proteolytic release of the cytotoxic agent is that the agent is typically attenuated when conjugated and the serum stabilities of the conjugates are typically high.

In other embodiments, the cleavable linker is pH-sensitive, i.e., sensitive to hydrolysis at certain pH values. Typically, the pH-sensitive linker is hydrolysable under acidic conditions. For example, an acid-labile linker that is hydrolysable in the lysosome (e.g., a hydrazone, semicarbazone, thiosemicarbazone, cis-aconitic amide, orthoester, acetal, ketal, or the like) can be used. Such linkers are relatively stable under neutral pH conditions, such as those in the blood, but are unstable at below pH 5.5 or 5.0, the approximate pH of the lysosome. In certain embodiments, the hydrolysable linker is a thioether linker (such as, e.g., a thioether attached to the therapeutic agent via an acylhydrazone bond).

In yet other embodiments, the linker may be cleaved under reducing conditions (e.g., a disulfide linker). A variety of disulfide linkers are known in the art, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), and SMPT (N-succinimidyl-oxycarbonyl-alpha-methyl-alpha-(2-pyridyl-dithio)toluene).

In certain preferred embodiments, the linker unit may have the following general formula:

-(T)ₐ-(W)_{w}-(Y)_{y}-

wherein:
T is a stretcher unit;
a is 0 or 1;
W is an amino acid unit;
w is an integer ranging from 0 to 12, preferably an integer ranging from 2 to 12;
Y is a spacer unit;
y is 0, 1 or 2.

The stretcher unit (T), when present, links the antibody to an amino acid unit (W) when present, or to the spacer unit when present, or directly to the drug. Useful functional groups that can be present on the antibody, either naturally or via chemical manipulation, include sulfhydryl, amino, hydroxyl, the anomeric hydroxyl group of a carbohydrate, and carboxyl. Suitable functional groups are sulfhydryl and amino. Sulfhydryl groups can be generated by reduction of the intramolecular disulfide bonds of the antibody, if present. Alternatively, sulfhydryl groups can be generated by reaction of an amino group of a lysine moiety of the antibody with 2-iminothiolane or other sulfhydryl generating reagents. In specific embodiments, the antibody is engineered to carry one or more lysines. More preferably, the antibody can be engineered to carry one or more Cysteines (cf. ThioMabs).

In certain specific embodiments, the stretcher unit forms a bond with a sulfur atom of the antibody. The sulfur atom can be derived from a sulfhydryl (-SH) group of a reduced antibody.

In certain other specific embodiments, the stretcher unit is linked to the antibody via a disulfide bond between a sulfur atom of the antibody and a sulfur atom of the stretcher unit.

In other specific embodiments, the reactive group of the stretcher contains a reactive site that can be reactive to an amino group of the antibody. The amino group can be that of an arginine or a lysine. Suitable amine reactive sites include, but are not limited to, activated esters (such as succinimide esters, 4-nitrophenyl esters, pentafluorophenyl esters), anhydrides, acid chlorides, sulfonyl chlorides, isocyanates and isothiocyanates.

In yet another aspect, the reactive function of the stretcher contains a reactive site that is reactive to a modified carbohydrate group that can be present on the antibody. In a specific embodiment, the antibody is glycosylated enzymatically to provide a carbohydrate moiety or is naturally glycosylated. The carbohydrate may be mildly oxidized with a reagent such as sodium periodate and the resulting carbonyl unit of the oxidized carbohydrate can be condensed with a stretcher that contains a functionality such as a hydrazide, an oxime, a reactive amine, a hydrazine, a thiosemicarbazide, a hydrazine carboxylate, or an arylhydrazide.

According to a particular embodiment, the stretcher unit has the following formula: 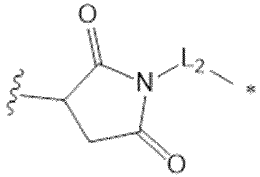 wherein
L₂ is (C₄-C₁₀)cycloalkyl-carbonyl, (C₂-C₆)alkyl or (C₂-C₆)alkyl-carbonyl (the cycloalkyl or alkyl moieties being linked to the nitrogen atom of the maleimide moiety),
the asterisk indicates the point of attachment to the amino acid unit, if present, to the spacer unit, if present, or to the drug D, and
the wavy line indicates the point of attachment to the antibody Ab.
By "(C₄-C₁₀)cycloalkyl" in the present invention is meant a hydrocarbon cycle having 4 to 10 carbon atoms including, but not limited to, cyclopentyl, cyclohexyl and the like.
L₂ can be advantageously (C₂-C₆)alkyl-carbonyl such as a pentyl-carbonyl of the following formula: 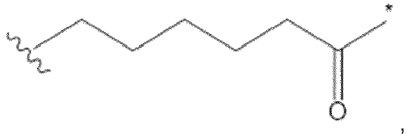 or
an ethylcarbonyl of the following formula: 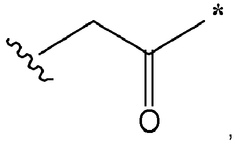 wherein
   the asterisk indicates the point of attachment to the amino acid unit, if present, to the spacer unit, if present, or to the drug D; and
   the wavy line indicates the point of attachment to the nitrogen atom of the maleimide moiety.

The amino acid unit (W), when present, links the stretcher unit (T) if present, or otherwise the antibody to the spacer unit (Y) if the spacer unit is present, or to the drug if the spacer unit is absent.

As above mentioned, (W)_{w} is absent (w = 0) or may be a dipeptide, tripeptide, tetrapeptide, pentapeptide, hexapeptide, heptapeptide, octapeptide, nonapeptide, decapeptide, undecapeptide or dodecapeptide unit, wherein the amino acids forming the peptides can be different from one another.

Thus (W)_{w} can be represented by the following formula: (W1)_{w1}(W2)_{w2}(W3)_{w3}(W4)_{w4}(W5)_{w5}, wherein each W1 to W5 represents, independently from one another, an amino acid unit and each w1 to w5 is 0 or 1.

In some embodiments, the amino acid unit (W)_{w} may comprise amino acid residues such as those occurring naturally, as well as minor amino acids and non-naturally occurring amino acid analogs, such as citrulline.

The amino acid residues of the amino acid unit (W)_{w} include, without limitation, alanine, valine, leucine, isoleucine, methionine, phenylalanine, tryptophan, proline, lysine protected or not with acetyl or formyl, arginine, arginine protected or not with tosyl or nitro groups, histidine, ornithine, ornithine protected with acetyl or formyl, and citrulline. Exemplary amino acid linker components include preferably a dipeptide, a tripeptide, a tetrapeptide or a pentapeptide, notably a dipeptide or a tripeptide.

Exemplary dipeptides include: Val-Cit, Ala-Val, Ala-Ala, Val-Ala, Lys-Lys, Cit-Cit, Val-Lys, Ala-Phe, Phe-Lys, Ala-Lys, Phe-Cit, Leu-Cit, Ile-Cit, Trp-Cit, Phe-Ala, Phe-N⁹-tosyl-Arg, Phe-N⁹-Nitro-Arg.

Exemplary tripeptides include: Val-Ala-Val, Ala-Asn-Val, Val-Leu-Lys, Ala-Ala-Asn, Phe-Phe-Lys, Gly-Gly-Gly, D-Phe-Phe-Lys, Gly-Phe-Lys.

Exemplary tetrapeptide include: Gly-Phe-Leu-Gly (SEQ ID NO. 79), Ala-Leu-Ala-Leu (SEQ ID NO. 80), Gly-Gly-Phe-Gly (SEQ ID NO. 81).

Exemplary pentapeptide include: Pro-Val-Gly-Val-Val (SEQ ID NO. 82).

According to a particular embodiment, (W)_{w} can be a dipeptide (i.e. w = 2) such as Val-Cit, Ala-Ala, or Val-Ala, a tetrapeptide such as Gly-Gly-Phe-Gly, or the linker lacks an amino acid unit (w=0, i.e. (W)_{w} is a single bond). When the linker lacks an amino acid unit, preferably it lacks also a spacer unit.

According to a preferred embodiment, w = 2 (i.e. (W)_{w} is a dipeptide) and (W)_{w} can thus be selected from: 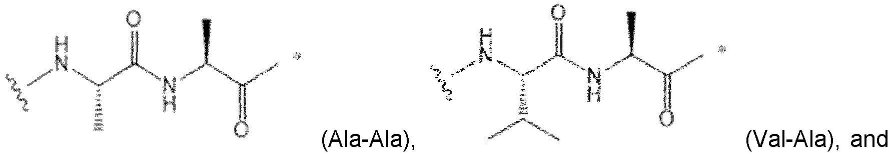 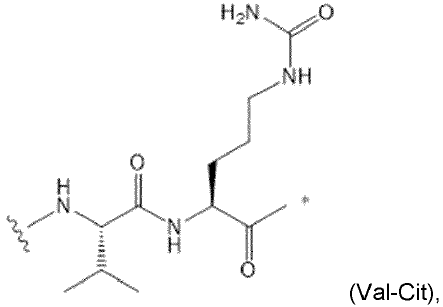 wherein
the asterisk indicates the point of attachment to the spacer unit if present, or to the drug D; and
the wavy line indicates the point of attachment to L₂.

Amino acid linker components can be designed and optimized in their selectivity for enzymatic cleavage by a particular enzyme, for example, a tumor-associated protease, cathepsin B, C and D, or a plasmin protease.

The amino acid unit of the linker can be enzymatically cleaved by an enzyme including, but not limited to, a tumor-associated protease to liberate the drug.

The amino acid unit can be designed and optimized in its selectivity for enzymatic cleavage by a particular tumor-associated protease. The suitable units are those whose cleavage is catalyzed by the proteases, cathepsin B, C and D, and plasmin.

The spacer unit (Y), when present, links an amino acid unit if present, or the stretcher unit if present, or otherwise the antibody to the drug. Spacer units are of two general types: self-immolative and non self-immolative. A non self-immolative spacer unit is one in which part or all of the spacer unit remains bound to the drug after enzymatic cleavage of an amino acid unit from the antibody-drug conjugate. Examples of a non self-immolative spacer unit include, but are not limited to a (glycine-glycine) spacer unit and a glycine spacer unit. To liberate the drug, an independent hydrolysis reaction should take place within the target cell to cleave the glycine-drug unit bond.

In a particular embodiment, a non self-immolative the spacer unit (Y) is Gly.

Alternatively, an antibody-drug conjugate containing a self-immolative spacer unit can release the drug without the need for a separate hydrolysis step. In these embodiments, (Y) is a residue of p-aminobenzyl alcohol (PAB) unit that is linked to (W)_{w} via the nitrogen atom of the PAB group, and connected directly to the drug via a ester, carbonate, carbamate or ether group.

Other examples of self-immolative spacers include, but are not limited to, aromatic compounds that are electronically equivalent to the PAB group such as residues of 2-aminoimidazol-5-methanol derivatives and ortho or para-aminobenzylacetals. Spacers can be used that undergo facile cyclization upon amide bond hydrolysis, such as substituted and unsubstituted 4-aminobutyric acid amides, appropriately substituted bicyclo[2.2.1] and bicyclo[2.2.2] ring systems and 2-aminophenylpropionic acid amides.

In an alternate embodiment, the spacer unit is a branched bis(hydroxymethyl)styrene (BHMS) unit, which can be used to incorporate additional drugs.

In a particular embodiment, the linker lacks a spacer unit (y = 0).

In another particular embodiment, the spacer unit (Y) is PAB-carbonyl with PAB being 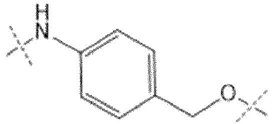 (the oxygen of the PAB unit being linked to the carbonyl), and y = 1.

In another particular embodiment, the spacer unit (Y) has the following formula: 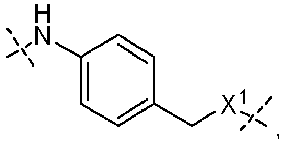 with X¹ = bond, O or N, preferably N, and y = 1.

In another particular embodiment, the spacer unit (Y) has the formula -NH-CH₂-, and y = 1.

In a particular embodiment, the linker has the following formula (III): 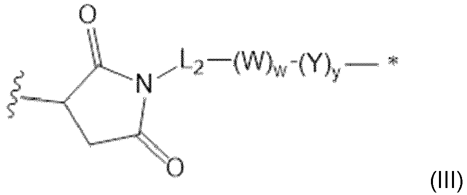 wherein
L₂ is (C₄-C₁₀)cycloalkyl-carbonyl, (C₂-C₆)alkyl or (C₂-C₆)alkyl-carbonyl (the carbonyl of these moieties, when present, being linked to (W)_{w}),
w is 0 and W is absent or w is an integer from 1 to 5, preferably 2, and W represents an amino acid unit,
y is 0 and Y is absent or y is 1 and Y is:
   PAB-carbonyl, with PAB being 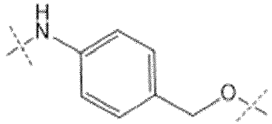 (the oxygen of the PAB unit being linked to the carbonyl),
   a group of the following formula: 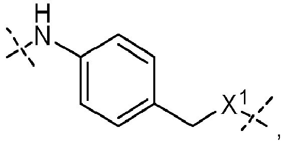 with X¹ = bond, O or N, preferably N, or a group of formula -NH-CH₂-,
   the asterisk indicates the point of attachment to the drug **D,** and
   the wavy line indicates the point of attachment to the antibody **Ab.**

Preferably y is 0 when w is 0 and y is 0 or 1 when w is an integer from 1 to 5.

Advantageously, L₂ is (C₂-C₆)alkyl-carbonyl such as a pentyl-carbonyl of the following formula: 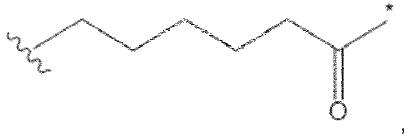 or an ethylcarbonyl of the following formula: 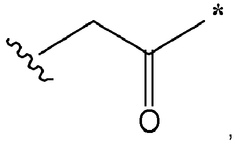 wherein
the asterisk indicates the point of attachment to (W)_{w}; and
the wavy line indicates the point of attachment to the nitrogen atom of the maleimide moiety.

According to an embodiment, **L** is selected from: 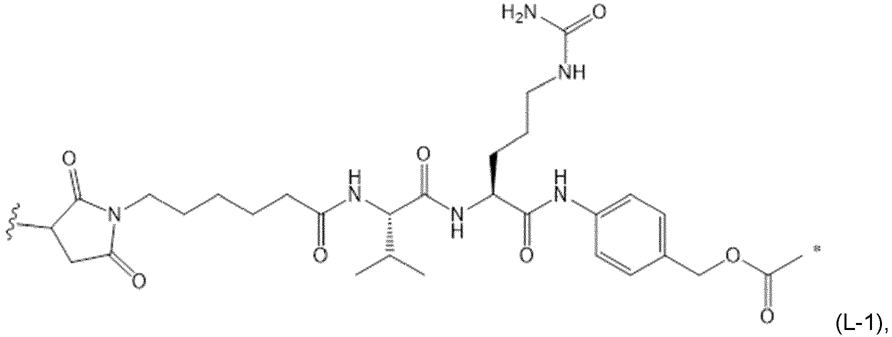 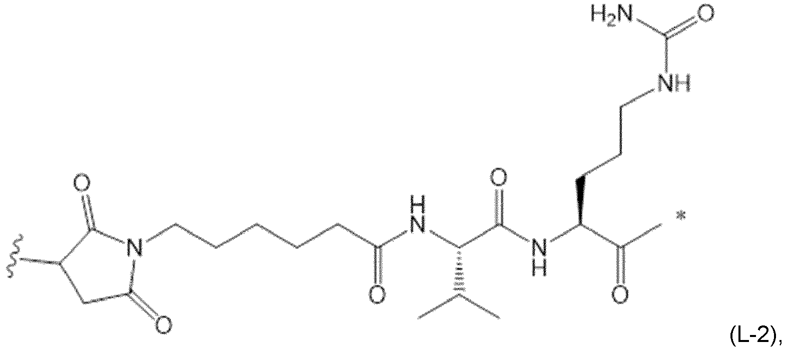 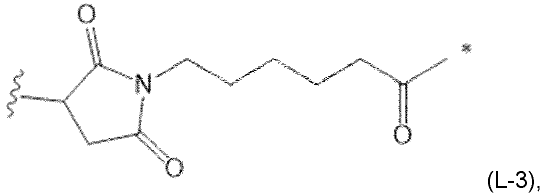 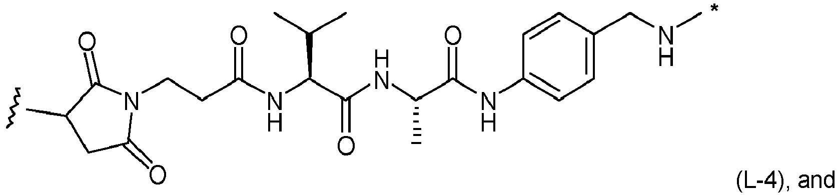 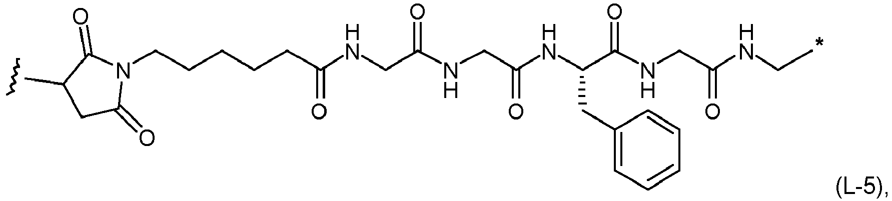 wherein the asterisk indicates the point of attachment to **D,** and the wavy line indicates the point of attachment to **Ab.**

According to a particular embodiment, **L-D** has the following formula (IV):

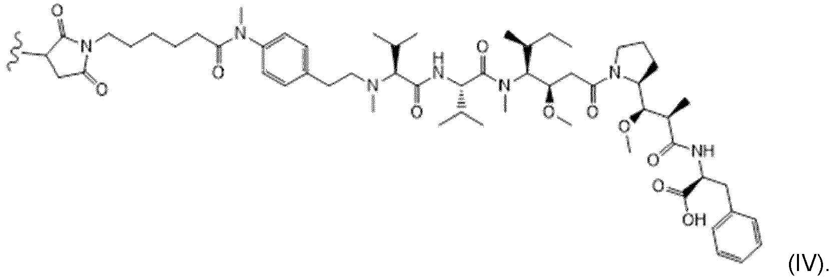

According to another particular embodiment, the ADC according to the invention has the following formula (V): 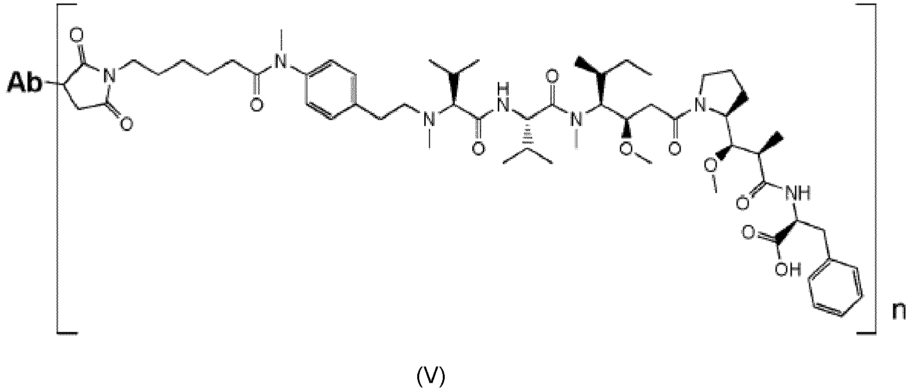 wherein **Ab** is an antibody, or an antigen binding fragment thereof as defined below, and preferably wherein **Ab** is an antibody, or an antigen binding fragment thereof, capable of binding to the human IGF-1R as defined below.

### Antibody

The terms "antibody", "antibodies" "ab", "Ab", "MAb" or "immunoglobulin" are used interchangeably in the broadest sense and include monoclonal antibodies, isolated, engineered or recombinant antibodies (e.g., full length or intact monoclonal antibodies), polyclonal antibodies, multivalent antibodies or multispecific antibodies (e.g., bispecific antibodies) and also antibody fragment thereof, so long as they exhibit the desired biological activity.

In an embodiment, the antibody of the ADC of the invention consists of a recombinant antibody. The term "recombinant antibody" refers to an antibody that results from the expression of recombinant DNA within living cells. A recombinant antibody of ADC of the invention is obtained by using laboratory methods of genetic recombination, well known by a person skilled in the art, creating DNA sequences that would not be found in biological organisms.

In another embodiment, the antibody of the ADC of the invention consists of a chemically synthesized antibody.

More particularly, such a molecule consists of a glycoprotein comprising at least two heavy (H) chains and two light (L) chains inter-connected by disulfide bonds. Each heavy chain comprises a heavy chain variable region (or domain) (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g. effector cells) and the first component (Clq) of the classical complement system.

By "antigen binding fragment" of an antibody of the ADC according to the invention, it is intended to indicate any peptide, polypeptide, or protein retaining the ability to bind to the target (also generally referred as antigen) of the antibody.

In an embodiment, such "antigen binding fragments" are selected in the group consisting of Fv, scFv (sc for single chain), Fab, F(ab')₂, Fab', scFv-Fc fragments or diabodies, or any fragment of which the half-life time would have been increased by chemical modification, such as the addition of poly(alkylene) glycol such as poly(ethylene) glycol ("PEGylation") (pegylated fragments called Fv-PEG, scFv-PEG, Fab-PEG, F(ab')₂-PEG or Fab'-PEG) ("PEG" for Poly(Ethylene) Glycol), or by incorporation in a liposome, said fragments having at least one of the characteristic CDRs of the antibody according to the invention. Preferably, said "antigen binding fragments" will be constituted or will comprise a partial sequence of the heavy or light variable chain of the antibody from which they are derived, said partial sequence being sufficient to retain the same specificity of binding as the antibody from which it is descended and a sufficient affinity, preferably at least equal to 1/100, in a more preferred manner to at least 1/10, of the affinity of the antibody from which it is descended, with respect to the target. More preferably, said "antigen binding fragments" will be constituted of or will comprise at least the three CDRs CDR-H 1, CDR-H2 and CDR-H3 of the heavy variable chain and the three CDRs CDR-L1, CDR-L2 and CDR-L3 of the light variable chain of the antibody from which they are derived.

By "binding", "binds", or the like, it is intended that the antibody, or any antigen binding fragment thereof, forms a complex with an antigen that is relatively stable under physiologic conditions. Specific binding can be characterized by an equilibrium dissociation constant of at least about 1×10⁻⁶ M. Methods for determining whether two molecules bind are well known in the art and include, for example, equilibrium dialysis, surface plasmon resonance, radiolabelled assays and the like. For the avoidance of doubt, it does not mean that the said antibody could not bind or interfere, at a low level, to another antigen. Nevertheless, as an embodiment, the said antibody binds only to the said antigen.

The term "epitope" is a region of an antigen that is bound by an antibody. Epitopes may be defined as structural or functional. Functional epitopes are generally a subset of the structural epitopes and have those residues that directly contribute to the affinity of the interaction. Epitopes may also be conformational, that is, composed of nonlinear amino acids. In certain embodiments, epitopes may include determinants that are chemically active surface groupings of molecules such as amino acids, sugar side chains, phosphoryl groups, or sulfonyl groups, and, in certain embodiments, may have specific three-dimensional structural characteristics, and/or specific charge characteristics.

Important keys to success with ADC therapy are thought to be the target antigen specificity and the internalization of the antigen-antibody complexes into the cancer cells. Obviously non-internalizing antigens are less effective than internalizing antigens to delivers cytotoxic agents. Internalization processes are variable across antigens and depend on multiple parameters that can be influenced by antibodies.

In the ADC, the cytotoxic confers the cytotoxic activity and the used antibody is responsible for the specificity against cancer cells, as well as a vector for entering within the cells to correctly address the cytotoxic. Thus to improve the ADC, the antibody can exhibit high ability to internalize into the targeted cancer cells. The efficiency of the antibody mediated internalisation differs significantly depending on the epitope targeted.

In an embodiment, the internalization of the antibody of the ADC according to the invention can be evaluated by immunofluorescence or FACS (Flow Cytometry) (as exemplified in WO2015162291 and WO2015162292) or any method or process known by the person skilled in the art specific for the internalization mechanism. In a preferred embodiment, the antibody of the ADC according to the invention can induce internalization after binding to the antigen of at least 30%, preferentially 50% and more preferentially 80%.

It is desirable that the used antibodies exhibit a rapid rate of internalization, preferably within 24 hours from administration of the antibody and, more preferably within 12 hours and, even more preferably within 6 hours.

According to a particular embodiment, the antibody or **Ab** is an antibody, or an antigen binding fragment thereof, capable of binding to IGF-1R (e.g. human IGF-1R).
According to a particular embodiment, the antibody or **Ab** is capable of binding to the human IGF-IR and is an antibody, or an antigen binding fragment thereof, comprising the three heavy chain CDRs of sequence SEQ ID No. 1, 2 and 3 and the three light chain CDRs of sequence SEQ ID No. 4, 5 and 6.

According to the invention, "an antibody, or an antigen binding fragment thereof, capable of binding to IGF-1R" means an antibody, or an antigen binding fragment thereof, capable of binding to IGF-1R, in particular to an epitope localized into IGF-1R, preferably the IGF-1R extracellular domain, more preferably the human IGF-1R (SEQ ID No. 50) and still more preferably the human IGF-1R extracellular domain (SEQ ID No. 51), and still more preferably to the N-terminal of the human IGF-1R extracellular domain (SEQ ID No. 52), or any natural variant sequence thereof.

| **SEQ ID No. 50:** |
|---|
| MKSGSGGGSPTSLWGLLFLSAALSLWPTSGEICGPGIDIRNDYQQLKRLENCTVIEGYLHILLISKAEDYRSY RFPKLTVITEYLLLFRVAGLESLGDLFPNLTVIRGWKLFYNYALVIFEMTNLKDIGLYNLRNITRGAIRIEKNA DLCYLSTVDWSLILDAVSNNYIVGNKPPKECGDLCPGTMEEKPMCEKTTINNEYNYRCWTTNRCQKMC PSTCGKRACTENNECCHPECLGSCSAPDNDTACVACRHYYYAGVCVPACPPNTYRFEGWRCVDRDFCA NILSAESSDSEGFVIHDGECMQECPSGFIRNGSQSMYCIPCEGPCPKVCEEEKKTKTIDSVTSAQMLQGCT IFKGNLLINIRRGNNIASELENFMGLIEVVTGYVKIRHSHALVSLSFLKNLRLILGEEQLEGNYSFYVLDNQNL QQLWDWDHRNLTIKAGKMYFAFNPKLCVSEIYRMEEVTGTKGRQSKGDINTRNNGERASCESDVLHFT STTTSKNRIIITWHRYRPPDYRDLISFTVYYKEAPFKNVTEYDGQDACGSNSWNMVDVDLPPNKDVEPGI LLHGLKPWTQYAVYVKAVTLTMVENDHIRGAKSEILYIRTNASVPSIPLDVLSASNSSSQLIVKWNPPSLP NGNLSYYIVRWQRQPQDGYLYRHNYCSKDKIPIRKYADGTIDIEEVTENPKTEVCGGEKGPCCACPKTEA EKQAEKEEAEYRKVFENFLHNSIFVPRPERKRRDVMQVANTTMSSRSRNTTAADTYNITDPEELETEYPFF ESRVDNKERTVISNLRPFTLYRIDIHSCNHEAEKLGCSASNFVFARTMPAEGADDIPGPVTWEPRPENSIFL KWPEPENPNGLILMYEIKYGSQVEDQRECVSRQEYRKYGGAKLNRLNPGNYTARIQATSLSGNGSWTDP VFFYVQAKTGYENFIHLIIALPVAVLLIVGGLVIMLYVFHRKRNNSRLGNGVLYASVNPEYFSAADVYVPDE WEVAREKITMSRELGQGSFGMVYEGVAKGVVKDEPETRVAIKTVNEAASMRERIEFLNEASVMKEFNC HHVVRLLGVVSQGQPTLVIMELMTRGDLKSYLRSLRPEMENNPVLAPPSLSKMIQMAGEIADGMAYLN ANKFVHRDLAARNCMVAEDFTVKIGDFGMTRDIYETDYYRKGGKGLLPVRWMSPESLKDGVFTTYSDV WSFGVVLWEIATLAEQPYQGLSNEQVLRFVMEGGLLDKPDNCPDMLFELMRMCWQYNPKMRPSFLEI ISSIKEEMEPGFREVSFYYSEENKLPEPEELDLEPENMESVPLDPSASSSLPLPDRHSGHKAENGPGPGVL VLRASFDERQPYAHMNGGRKNERALPLPQSSTC |

| **SEQ ID No. 51:** |
|---|
| MKSGSGGGSPTSLWGLLFLSAALSLWPTSGEICGPGIDIRNDYQQLKRLENCTVIEGYLHILLISKAEDYRSY RFPKLTVITEYLLLFRVAGLESLGDLFPNLTVIRGWKLFYNYALVIFEMTNLKDIGLYNLRNITRGAIRIEKNA DLCYLSTVDWSLILDAVSNNYIVGNKPPKECGDLCPGTMEEKPMCEKTTINNEYNYRCWTTNRCQKMC PSTCGKRACTENNECCHPECLGSCSAPDNDTACVACRHYYYAGVCVPACPPNTYRFEGWRCVDRDFCA NILSAESSDSEGFVIHDGECMQECPSGFIRNGSQSMYCIPCEGPCPKVCEEEKKTKTIDSVTSAQMLQGCT IFKGNLLINIRRGNNIASELENFMGLIEVVTGYVKIRHSHALVSLSFLKNLRLILGEEQLEGNYSFYVLDNQNL QQLWDWDHRNLTIKAGKMYFAFNPKLCVSEIYRMEEVTGTKGRQSKGDINTRNNGERASCESDVLHFT STTTSKNRIIITWHRYRPPDYRDLISFTVYYKEAPFKNVTEYDGQDACGSNSWNMVDVDLPPNKDVEPGI LLHGLKPWTQYAVYVKAVTLTMVENDHIRGAKSEILYIRTNASVPSIPLDVLSASNSSSQLIVKWNPPSLP NGNLSYYIVRWQRQPQDGYLYRHNYCSKDKIPIRKYADGTIDIEEVTENPKTEVCGGEKGPCCACPKTEA EKQAEKEEAEYRKVFENFLHNSIFVPRPERKRRDVMQVANTTMSSRSRNTTAADTYNITDPEELETEYPFF ESRVDNKERTVISNLRPFTLYRIDIHSCNHEAEKLGCSASNFVFARTMPAEGADDIPGPVTWEPRPENSIFL KWPEPENPNGLILMYEIKYGSQVEDQRECVSRQEYRKYGGAKLNRLNPGNYTARIQATSLSGNGSWTDP VFFYVQAKTGYEN |

| **SEQ ID No. 52:** |
|---|
| MKSGSGGGSPTSLWGLLFLSAALSLWPTSGEICGPGIDIRNDYQQLKRLENCTVIEGYLHILLISKAEDYRSY RFPKLTVITEYLLLFRVAGLESLGDLFPNLTVIRGWKLFYNYALVIFEMTNLKDIGLYNLRNITRGAIRIEKNA DLCYLSTVDWSLILDAVSNNYIVGNKPPKECGDLCPGTMEEKPMCEKTTINNEYNYRCWTTNRCQKMC PSTCGKRACTENNECCHPECLGSCSAPDNDTACVACRHYYYAGVCVPACPPNTYRFEGWRCVDRDFCA NILSAESSDSEGFVIHDGECMQECPSGFIRNGSQSMYCIPCEGPCPKVCEEEKKTKTIDSVTSAQMLQGCT IFKGNLLINIRRGNNIASELENFMGLIEVVTGYVKIRHSHALVSLSFLKNLRLILGEEQLEGNYSFYVLDNQNL QQLWDWDHRNLTIKAGKMYFAFNPKLCVSEIYRMEEVTGTKGRQSKGDINTRNNGERASCESDVLHFT STTTSKNRIIITWHRY |

The IMGT unique numbering has been defined to compare the variable domains whatever the antigen receptor, the chain type, or the species [Lefranc M.-P., Immunology Today 18, 509 (1997) / Lefranc M.-P., The Immunologist, 7, 132-136 (1999) / Lefranc, M.-P., Pommié, C., Ruiz, M., Giudicelli, V., Foulquier, E., Truong, L., Thouvenin-Contet, V. and Lefranc, Dev. Comp. Immunol., 27, 55-77 (2003)]. In the IMGT unique numbering, the conserved amino acids always have the same position, for instance cystein 23 (1st-CYS), tryptophan 41 (CONSERVED-TRP), hydrophobic amino acid 89, cystein 104 (2nd-CYS), phenylalanine or tryptophan 118 (J-PHE or J-TRP). The IMGT unique numbering provides a standardized delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117. As gaps represent unoccupied positions, the CDR-IMGT lengths (shown between brackets and separated by dots, e.g. [8.8.13]) become crucial information. The IMGT unique numbering is used in 2D graphical representations, designated as IMGT Colliers de Perles [Ruiz, M. and Lefranc, M.-P., Immunogenetics, 53, 857-883 (2002) / Kaas, Q. and Lefranc, M.-P., Current Bioinformatics, 2, 21-30 (2007)], and in 3D structures in IMGT/3Dstructure-DB [Kaas, Q., Ruiz, M. and Lefranc, M.-P., T cell receptor and MHC structural data. Nucl. Acids. Res., 32, D208-D210 (2004)].

It must be understood that, without contradictory specification in the present specification, complementarity-determining regions or CDRs, mean the hypervariable regions of the heavy and light chains of immunoglobulins as defined according to the IMGT numbering system.

Nevertheless, CDRs can also be defined according to the Kabat numbering system (Kabat et al., Sequences of proteins of immunological interest, 5th Ed., U.S. Department of Health and Human Services, NIH, 1991, and later editions). There are three heavy chain CDRs and three light chain CDRs. Here, the terms "CDR" and "CDRs" are used to indicate, depending on the case, one or more, or even all, of the regions containing the majority of the amino acid residues responsible for the antibody's binding affinity for the antigen or epitope it recognizes. In order to simplify the reading of the present application, the CDRs according to Kabat are not defined. Nevertheless, it would be obvious for the person skilled in the art, using the definition of the CDRs according to IMGT, to define the CDRs according to Kabat.

According to an embodiment, **Ab** comprises the three heavy chain CDRs comprising or consisting of the sequences SEQ ID Nos. 1, 2 and 3, or any sequence exhibiting at least 80%, preferably 85%, 90%, 95% and 98% identity with SEQ ID Nos. 1, 2 or 3; and the three light chain CDRs comprising or consisting of the sequences SEQ ID Nos. 4, 5 and 6, or any sequence exhibiting at least 80%, preferably 85%, 90%, 95% and 98% identity with SEQ ID Nos. 4, 5 or 6.

In the sense of the present invention, the "identity" or "percentage identity" between two sequences of nucleic acids or amino acids means the percentage of identical nucleotides or amino acid residues between the two sequences to be compared, obtained after optimal alignment, this percentage being purely statistical and the differences between the two sequences being distributed randomly along their length. The comparison of two nucleic acid or amino acid sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981) [Ad. App. Math. 2:482], by means of the local homology algorithm of Neddleman and Wunsch (1970) [J. Mol. Biol. 48:443], by means of the similarity search method of Pearson and Lipman (1988) [Proc. Natl. Acad. Sci. USA 85:2444] or by means of computer software using these algorithms (GAP, BESTFIT, FASTA and TFASTA in the Wisconsin Genetics Software Package, Genetics Computer Group, 575 Science Dr., Madison, WI, or by the comparison software BLAST NR or BLAST P).

Percentage identity is calculated by determining the number of positions at which the amino acid nucleotide or residue is identical between the two sequences, preferably between the two complete sequences, dividing the number of identical positions by the total number of positions in the alignment window and multiplying the result by 100 to obtain the percentage identity between the two sequences.

For example, the BLAST program, "BLAST 2 sequences" (Tatusova et al., "Blast 2 sequences - a new tool for comparing protein and nucleotide sequences", FEMS Microbiol., 1999, Lett. 174:247-250) available on the site http://www.ncbi.nlm.nih.gov/gorf/bl2.html, can be used with the default parameters (notably for the parameters "open gap penalty": 5, and "extension gap penalty": 2; the selected matrix being for example the "BLOSUM 62" matrix proposed by the program); the percentage identity between the two sequences to compare is calculated directly by the program.

For the amino acid sequence exhibiting at least 80%, preferably 85%, 90%, 95% and 98% identity with a reference amino acid sequence, preferred examples include those containing the reference sequence, certain modifications, notably a deletion, addition or substitution of at least one amino acid, truncation or extension. In the case of substitution of one or more consecutive or non-consecutive amino acids, substitutions are preferred in which the substituted amino acids are replaced by "equivalent" amino acids. Here, the expression "equivalent amino acids" is meant to indicate any amino acids likely to be substituted for one of the structural amino acids without however modifying the biological activities of the corresponding antibodies and of those specific examples defined below.

Equivalent amino acids can be determined either on their structural homology with the amino acids for which they are substituted or on the results of comparative tests of biological activity between the various antibodies likely to be generated.

As a non-limiting example, Table 1 below summarizes the possible substitutions likely to be carried out without resulting in a significant modification of the biological activity of the corresponding modified antibody; inverse substitutions are naturally possible under the same conditions.

**Table 1**

| Original residue | Substitution(s) |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (E) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

For more clarity, the following Tables 2a and 2b illustrate the CDR sequences, defined according to IMGT, for the preferred antibodies.

**Table 2a**

| | **Heavy chain** | **Light chain** | **SEQ ID No.** |
|---|---|---|---|
| Consensus | CDR-H 1 | | 1 |
| | CDR-H2 | | 2 |
| | CDR-H3 | | 3 |
| | | CDR-L 1 | 4 |
| | | CDR-L2 | 5 |
| | | CDR-L3 | 6 |
| 208F2 | CDR-H 1 | | 7 |
| | CDR-H2 | | 2 |
| | CDR-H3 | | 3 |
| | | CDR-L 1 | 9 |
| | | CDR-L2 | 5 |
| | | CDR-L3 | 11 |
| 212A11 | CDR-H 1 | | 7 |
| | CDR-H2 | | 2 |
| | CDR-H3 | | 3 |
| | | CDR-L 1 | 10 |
| | | CDR-L2 | 5 |
| | | CDR-L3 | 11 |
| 214F8 & 213B10 | CDR-H 1 | | 7 |
| | CDR-H2 | | 2 |
| | CDR-H3 | | 3 |
| | | CDR-L 1 | 9 |
| | | CDR-L2 | 5 |
| | | CDR-L3 | 12 |
| 219D6 | CDR-H 1 | | 8 |
| | CDR-H2 | | 2 |
| | CDR-H3 | | 3 |
| | | CDR-L 1 | 9 |
| | | CDR-L2 | 5 |
| | | CDR-L3 | 11 |

**Table 2b**

| SEQ ID No. | Sequence |
|---|---|
| 1 | GYTFTSYY |
| | (position 3: T may be replaced by S; position 8: Y may be replaced by F) |
| 2 | IWPGDGST |
| 3 | ASPMITPNYAMDY |
| 4 | QDISKY |
| | (position 4: S may be replaced by N) |
| 5 | YTS |
| 6 | QQGSTLPYT |
| | (position 5: T may be replaced by A) |
| 7 | GYTFTSYY |
| 8 | GYSFTSYF |
| 9 | QDISKY |
| 10 | QDINKY |
| 11 | QQGSTLPYT |
| 12 | QQGSALPYT |

In still another embodiment, the antibody of the ADC of the invention consists of a monoclonal antibody.

The term "monoclonal antibody" or "Mab" as used herein refers to an antibody obtained from a population of substantially homogeneous antibodies, i.e. the individual antibodies of the population are identical except for possible naturally occurring mutations that may be present in minor amounts. Monoclonal antibodies are highly specific, being directed against a single epitope. Such monoclonal antibody may be produced by a single clone of B cells or hybridoma. Monoclonal antibodies may also be recombinant, i.e. produced by protein engineering or chemical synthesis. Monoclonal antibodies may also be isolated from phage antibody libraries. In addition, in contrast with preparations of polyclonal antibodies which typically include various antibodies directed against various determinants, or epitopes, each monoclonal antibody is directed against a single epitope of the antigen.

The monoclonal antibody herein includes murine, chimeric and humanized antibody, such as described after.

According to a specific aspect, the antibody is a murine antibody characterized in that said antibody also comprises light chain and heavy chain constant regions derived from an antibody of a species heterologous with the mouse, notably man.

According to another specific aspect, the antibody is a chimeric (c) antibody characterized in that said antibody also comprises light chain and heavy chain constant regions derived from an antibody of a species heterologous with the mouse, notably human.

A chimeric antibody is one containing a natural variable region (light chain and heavy chain) derived from an antibody of a given species in combination with constant regions of the light chain and the heavy chain of an antibody of a species heterologous to said given species.

According to an embodiment, the antibody is selected from:
a) an antibody comprising the three heavy chain CDRs of sequence SEQ ID No. 7, 2 and 3 and the three light chain CDRs of sequence SEQ ID No. 9, 5 and 11;
b) an antibody comprising the three heavy chain CDRs of sequence SEQ ID No. 7, 2 and 3 and the three light chain CDRs of sequence SEQ ID No. 10, 5 and 11;
c) an antibody comprising the three heavy chain CDRs of sequence SEQ ID No. 7, 2 and 3 and the three light chain CDRs of sequence SEQ ID No. 9, 5 and 12; and
d) an antibody comprising the three heavy chain CDRs of sequence SEQ ID No. 8, 2 and 3 and the three light chain CDRs of sequence SEQ ID No. 9, 5 and 11.

According to a particular embodiment, the antibody is selected from:
a) an antibody comprising a heavy chain variable domain of sequence SEQ ID No. 13 or any sequence exhibiting at least 80% identity with SEQ ID No. 13 and the three light chain CDRs of sequences SEQ ID Nos. 9, 5 and 11;
b) an antibody comprising a heavy chain variable domain of sequence SEQ ID No. 14 or any sequence exhibiting at least 80% identity with SEQ ID No. 14 and the three light chain CDRs of sequences SEQ ID Nos. 10, 5 and 11;
c) an antibody comprising a heavy chain variable domain of sequence SEQ ID No. 15 or any sequence exhibiting at least 80% identity with SEQ ID No. 15 and the three light chain CDRs of sequences SEQ ID Nos. 9, 5 and 12;
d) an antibody comprising a heavy chain variable domain of sequence SEQ ID No. 16 or any sequence exhibiting at least 80% identity with SEQ ID No. 16 and the three light chain CDRs of sequences SEQ ID Nos. 9, 5 and 11; and
e) an antibody comprising a heavy chain variable domain of sequence SEQ ID No. 17 or any sequence exhibiting at least 80% identity with SEQ ID No. 17 and the three light chain CDRs of sequences SEQ ID Nos. 9, 5 and 12.

By "any sequence exhibiting at least 80%, preferably 85%, 90%, 95% and 98% identity with SEQ ID No. 13 to 17", its is intended to designate the sequences exhibiting the three heavy chain CDRs SEQ ID Nos. 1, 2 and 3 and, in addition, exhibiting at least 80%, preferably 85%, 90%, 95% and 98%, identity with the full sequence SEQ ID No. 13 to 17 outside the sequences corresponding to the CDRs (i.e. SEQ ID No. 1, 2 and 3).

According to an embodiment, the antibody is selected from:
a) an antibody comprising a heavy chain variable domain of sequence SEQ ID No. 13 and the three light chain CDRs of sequence SEQ ID No. 9, 5 and 11;
b) an antibody comprising a heavy chain variable domain of sequence SEQ ID No. 14 and the three light chain CDRs of sequence SEQ ID No. 10, 5 and 11;
c) an antibody comprising a heavy chain variable domain of sequence SEQ ID No. 15 and the three light chain CDRs of sequence SEQ ID No. 9, 5 and 12;
d) an antibody comprising a heavy chain variable domain of sequence SEQ ID No. 16 and the three light chain CDRs of sequence SEQ ID No. 9, 5 and 11; and
e) an antibody comprising a heavy chain variable domain of sequence SEQ ID No. 17 and the three light chain CDRs of sequence SEQ ID No. 9, 5 and 12.

According to an embodiment, the antibody is selected from:
a) an antibody comprising a light chain variable domain of sequence SEQ ID No. 18 or any sequence exhibiting at least 80% identity with SEQ ID No. 18 and the three heavy chain CDRs of sequences SEQ ID Nos. 7, 2 and 3;
b) an antibody comprising a light chain variable domain of sequence SEQ ID No. 19 or any sequence exhibiting at least 80% identity with SEQ ID No. 19 and the three heavy chain CDRs of sequences SEQ ID Nos. 7, 2 and 3;
c) an antibody comprising a light chain variable domain of sequence SEQ ID No. 20 or any sequence exhibiting at least 80% identity with SEQ ID No. 20 and the three heavy chain CDRs of sequences SEQ ID Nos. 7, 2 and 3;
d) an antibody comprising a light chain variable domain of sequence SEQ ID No. 21 or any sequence exhibiting at least 80% identity with SEQ ID No. 21 and the three heavy chain CDRs of sequences SEQ ID Nos. 8, 2 and 3; and
e) an antibody comprising a light chain variable domain of sequence SEQ ID No. 22 or any sequence exhibiting at least 80% identity with SEQ ID No. 22 and the three heavy chain CDRs of sequences SEQ ID Nos. 7, 2 and 3.

By "any sequence exhibiting at least 80%, preferably 85%, 90%, 95% and 98% identity with SEQ ID No. 18 to 22", it is intended to designate respectively the sequences exhibiting the three light chain CDRs SEQ ID Nos. 4, 5 and 6 and, in addition, exhibiting at least 80%, preferably 85%, 90%, 95% and 98%, identity with the full sequence SEQ ID No. 18 to 22 outside the sequences corresponding to the CDRs (i.e. SEQ ID No. 4, 5 and 6).

According to an embodiment, the antibody is selected from:
a) an antibody comprising a light chain variable domain of sequence SEQ ID No. 18 and the three heavy chain CDRs of sequence SEQ ID No. 7, 2 and 3;
b) an antibody comprising a light chain variable domain of sequence SEQ ID No. 19 and the three heavy chain CDRs of sequence SEQ ID No. 7, 2 and 3;
c) an antibody comprising a light chain variable domain of sequence SEQ ID No. 20 and the three heavy chain CDRs of sequence SEQ ID No. 7, 2 and 3;
d) an antibody comprising a light chain variable domain of sequence SEQ ID No. 21 and the three heavy chain CDRs of sequence SEQ ID No. 8, 2 and 3; and
e) an antibody comprising a light chain variable domain of sequence SEQ ID No. 22 and the three heavy chain CDRs of sequence SEQ ID No. 7, 2 and 3.

According to an embodiment, the antibody is selected from:
a) an antibody comprising a heavy chain variable domain of sequence SEQ ID No. 13 or any sequence exhibiting at least 80% identity with SEQ ID No. 13 and a light chain variable domain of sequence SEQ ID No. 18 or any sequence exhibiting at least 80% identity with SEQ ID No. 18;
b) an antibody comprising a heavy chain variable domain of sequence SEQ ID No. 14 or any sequence exhibiting at least 80% identity with SEQ ID No. 14 and a light chain variable domain of sequence SEQ ID No. 19 or any sequence exhibiting at least 80% identity with SEQ ID NO. 19;
c) an antibody comprising a heavy chain variable domain of sequence SEQ ID No. 15 or any sequence exhibiting at least 80% identity with SEQ ID No. 15 and a light chain variable domain of sequence SEQ ID No. 20 or any sequence exhibiting at least 80% identity with SEQ ID No. 20;
d) an antibody comprising a heavy chain variable domain of sequence SEQ ID No. 16 or any sequence exhibiting at least 80% identity with SEQ ID No. 16 and a light chain variable domain of sequence SEQ ID No. 21 or any sequence exhibiting at least 80% identity with SEQ ID No. 21; and
e) an antibody comprising a heavy chain variable domain of sequence SEQ ID No. 17 or any sequence exhibiting at least 80% identity with SEQ ID No. 17 and a light chain variable domain of sequence SEQ ID No. 22 or any sequence exhibiting at least 80% identity with SEQ ID No. 22.

Chimeric antibodies herein described can be also characterized by the constant domain and, more particularly, said chimeric antibodies can be selected or designed such as, without limitation, IgG1, IgG2, IgG3, IgM, IgA, IgD or IgE. More preferably, in the context of the present invention, said chimeric antibodies are IgG1 or IgG4.

According to an embodiment, the antibody is a chimeric antibody comprising variable domains VH and VL as above described in the format IgG1. More preferably, said chimeric antibody comprises a constant domain for the VH of sequence SEQ ID No. 43 and a Kappa domain for the VL of sequence SEQ ID No. 45.

According to an embodiment, the antibody is a chimeric antibody comprising variable domains VH and VL as above described in the format IgG4. More preferably, said chimeric antibody comprises a constant domain for the VH of sequence SEQ ID No. 44 and a Kappa domain for the VL of sequence SEQ ID No. 45.

According to an embodiment, the antibody is selected from:
a) an antibody comprising or consisting of a heavy chain of sequence SEQ ID No. 23 or any sequence exhibiting at least 80% identity with SEQ ID No. 23 and a light chain of sequence SEQ ID No. 28 or any sequence exhibiting at least 80% identity with SEQ ID No. 28;
b) an antibody comprising or consisting of a heavy chain of sequence SEQ ID No. 24 or any sequence exhibiting at least 80% identity with SEQ ID No. 24 and a light chain of sequence SEQ ID No. 29 or any sequence exhibiting at least 80% identity with SEQ ID No. 29;
c) an antibody comprising or consisting of a heavy chain of sequence SEQ ID No. 25 or any sequence exhibiting at least 80% identity with SEQ ID No. 25 and a light chain of sequence SEQ ID No. 30 or any sequence exhibiting at least 80% identity with SEQ ID No. 30;
d) an antibody comprising or consisting of a heavy chain of sequence SEQ ID No. 26 or any sequence exhibiting at least 80% identity with SEQ ID No. 26 and a light chain of sequence SEQ ID No. 31or any sequence exhibiting at least 80% identity with SEQ ID No. 31; and
e) an antibody comprising or consisting of a heavy chain of sequence SEQ ID No. 27 or any sequence exhibiting at least 80% identity with SEQ ID No. 27 and a light chain of sequence SEQ ID No. 32 or any sequence exhibiting at least 80% identity with SEQ ID No. 32.

For more clarity, the following Tables 3a and 3b illustrate the sequences of the VH and VL, respectively, for the preferred chimeric antibodies.

**Table 3a**

| | **Heavy Chain** | **Light chain** | **SEQ ID No.** |
|---|---|---|---|
| c208F2 | Variable domain (VH) | | 13 |
| | | Variable domain (VL) | 18 |
| | Full length | | 23 |
| | | Full length | 28 |
| c212A11 | Variable domain (VH) | | 14 |
| | | Variable domain (VL) | 19 |
| | Full length | | 24 |
| | | Full length | 29 |
| c214F8 | Variable domain (VH) | | 15 |
| | | Variable domain (VL) | 20 |
| | Full length | | 25 |
| | | Full length | 30 |
| c219D6 | Variable domain (VH) | | 16 |
| | | Variable domain (VL) | 21 |
| | Full length | | 26 |
| | | Full length | 31 |
| c213B10 | Variable domain (VH) | | 17 |
| | | Variable domain (VL) | 22 |
| | Full length | | 27 |
| | | Full length | 32 |

**Table 3b**

| SEQ ID No. | Sequence |
|---|---|
| 13 | QVQLQQSGPE LVKPGASVKM SCKASGYTFT SYYIYWVKQR PGQGLEWLGW IWPGDGSTKY NEKFKDKTTL TADKSSNTAY MFLSSLTSED SAVYFCASPM ITPNYAMDYW GQGASVTVSS |
| 14 | QVQLQQSGPE LVKPGASVKM SCKASGYTFT SYYIHWVKQR PGQGLEWIGW IWPGDGSTKY NEKFKGKTTL TADKSSSTAY MFLSSLTSED SAVYFCASPM ITPNYAMDYW GQGASVTVSS |
| 15 | QVQLQQSGSE LVKPGASVKL SCKASGYTFT SYYIHWVKQR PGQGLEWIGW IWPGDGSTKY NERFKGKTTL TADKSSSTAY MFLSSLTSED SAVYFCASPM ITPNYAMDYW GQGASVTVSS |
| 16 | QVQLQQSGPE LVKPGDSVKM SCKASGYSFT SYFIHWVKQR PGQGLEWIGW IWPGDGSTKY NEKFKGKTTL TTDKSSSTAY MFLNSLTSED SAVYFCASPM ITPNYAMDYW GQGASVTVSS |
| 17 | QVQLQQSGSE LVKPGASVKM SCKASGYTFT SYYIHWVKQR PGQGLEWIGW IWPGDGSTKY NERFKGKTTL TADKSSSTAY MFLSSLTSED SAVYFCASPM ITPNYAMDYW GQGASVTVSS |
| 18 | DIQMTQTTSS LSASLGDRVT ISCRASQDIS KYLNWYQQQP DGTIKLLIYY TSRLHSGVPS RFSGRGSGTD YSLTISNVEQ EDIATYFCQQ GSTLPYTFGG GTKLEIK |
| 19 | DIQMTQTTSS LSASLGDRVT ISCRASQDIN KYLNWYQQQP DGTVKLLIYY TSRLHSGVPS RFSGRGSGTD YSLTISNLEQ EDIATYFCQQ GSTLPYTFGG GTKLEIK |
| 20 | DIQMTQTTSS LSASLGDRVT FSCRASQDIS KYLNWYQQQP DGTIKLLIYY TSRLHSGVPS RFSGRGSGTD YSLTITNLEQ EDIATYFCQQ GSALPYTFGG GTKLEIK |
| 21 | DIQMTQTTSS LSASLGDRVT ISCRASQDIS KYLNWYQQQP DGTVKLLIYY TSRLHSGVPS RFSGRGSGTD YSLTISNLEQ EDIATYFCQQ GSTLPYTFGG GTKLEIK |
| 22 | DIQMTQTTSS LSASLGDRVT ISCRASQDIS KYLNWYQQQP DGTIKLLIYY TSRLHSGVPS RFSGRGSGTD YSLTITNLEQ EDIATYFCQQ GSALPYTFGG GTKLEIK |
| 23 | QVQLQQSGPE LVKPGASVKM SCKASGYTFT SYYIYWVKQR PGQGLEWLGW IWPGDGSTKY NEKFKDKTTL TADKSSNTAY MFLSSLTSED SAVYFCASPM ITPNYAMDYW GQGASVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 24 | QVQLQQSGPE LVKPGASVKM SCKASGYTFT SYYIHWVKQR PGQGLEWIGW IWPGDGSTKY NEKFKGKTTL TADKSSSTAY MFLSSLTSED SAVYFCASPM ITPNYAMDYW GQGASVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 25 | QVQLQQSGSE LVKPGASVKL SCKASGYTFT SYYIHWVKQR PGQGLEWIGW IWPGDGSTKY NERFKGKTTL TADKSSSTAY MFLSSLTSED SAVYFCASPM ITPNYAMDYW GQGASVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 26 | QVQLQQSGPE LVKPGDSVKM SCKASGYSFT SYFIHWVKQR PGQGLEWIGW IWPGDGSTKY NEKFKGKTTL TTDKSSSTAY MFLNSLTSED SAVYFCASPM ITPNYAMDYW GQGASVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 27 | QVQLQQSGSE LVKPGASVKM SCKASGYTFT SYYIHWVKQR PGQGLEWIGW IWPGDGSTKY NERFKGKTTL TADKSSSTAY MFLSSLTSED SAVYFCASPM ITPNYAMDYW GQGASVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 28 | DIQMTQTTSS LSASLGDRVT ISCRASQDIS KYLNWYQQQP DGTIKLLIYY TSRLHSGVPS RFSGRGSGTD YSLTISNVEQ EDIATYFCQQ GSTLPYTFGG GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 29 | DIQMTQTTSS LSASLGDRVT ISCRASQDIN KYLNWYQQQP DGTVKLLIYY TSRLHSGVPS RFSGRGSGTD YSLTISNLEQ EDIATYFCQQ GSTLPYTFGG GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 30 | DIQMTQTTSS LSASLGDRVT FSCRASQDIS KYLNWYQQQP DGTIKLLIYY TSRLHSGVPS RFSGRGSGTD YSLTITNLEQ EDIATYFCQQ GSALPYTFGG GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 31 | DIQMTQTTSS LSASLGDRVT ISCRASQDIS KYLNWYQQQP DGTVKLLIYY TSRLHSGVPS RFSGRGSGTD YSLTISNLEQ EDIATYFCQQ GSTLPYTFGG GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 32 | DIQMTQTTSS LSASLGDRVT ISCRASQDIS KYLNWYQQQP DGTIKLLIYY TSRLHSGVPS RFSGRGSGTD YSLTITNLEQ EDIATYFCQQ GSALPYTFGG GTKLEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |

According to another specific aspect of the present invention, the antibody is a humanized antibody characterized in that the constant regions of the light chain and the heavy chain derived from human antibody are, respectively, the lambda or kappa region and the gamma-1, gamma-2 or gamma-4 region.

"Humanized antibodies" means an antibody that contains CDR regions derived from an antibody of nonhuman origin, the other parts of the antibody molecule being derived from one (or several) human antibodies. In addition, some of the skeleton segment residues (called FR) can be modified to preserve binding affinity.

In a particular embodiment, the antibody comprises a heavy chain variable domain (VH) having:
i) the CDR-H1, CDR-H2 and CDR-H3 of sequences SEQ ID Nos. 7, 2 and 3, respectively, and
ii) the FR1, FR2 and FR3 derived from the human germline IGHV1-46*01 (SEQ ID No. 46), and
iii) the FR4 derived from the human germline IGHJ4*01 (SEQ ID No. 48).

In a particular embodiment, the antibody comprises a light chain variable domain (VL) having:
i) the CDR-L1, CDR-L2 and CDR-L3 of sequences SEQ ID Nos. 9, 5 and 11, respectively, and
ii) the FR1, FR2 and FR3 derived from the human germline IGKV1-39*01 (SEQ ID No. 47), and
iii) the FR4 derived from the human germline IGKJ4*01 (SEQ ID No. 49).

In a particular embodiment of the invention, the antibody comprises:
a) a heavy chain having CDR-H1, CDR-H2 and CDR-H3 of sequences SEQ ID Nos. 7, 2 and 3, respectively, and FR1, FR2 and FR3 derived from the human germline IGHV1-46*01 (SEQ ID No. 46), and the FR4 derived from the human germline IGHJ4*01 (SEQ ID No. 48); and
b) a light chain having CDR-L1, CDR-L2 and CDR-L3 of sequences SEQ ID Nos. 9, 5 and 11, respectively, and FR1, FR2 and FR3 derived from the human germline IGKV1-39*01 (SEQ ID No. 47), and the FR4 derived from the human germline IGKJ4*01 (SEQ ID No. 49).

In an embodiment, the antibody comprises a heavy chain variable domain (VH) of sequence SEQ ID No. 33 and a light chain variable domain (VL) of sequence SEQ ID No. 35. Said humanized antibody will be called thereinafter hz208F2 ("Variant 1" or "Var. 1").

In another embodiment, the antibody comprises a heavy chain variable domain (VH) of sequence SEQ ID No. 33 wherein said sequence SEQ ID No. 33 comprises at least 1 back-mutation selected from the residues 20, 34, 35, 38, 48, 50, 59, 61, 62, 70, 72, 74, 76, 77, 79, 82 and 95.

By the expressions "back-mutation" or "back mutation" it is meant a mutation or replacement of the human residue present in the germline by the corresponding residue initially present in the murine sequence.

In another embodiment, the antibody comprises a heavy chain variable domain (VH) of sequence SEQ ID No. 33 wherein said sequence SEQ ID No. 33 comprises 2, 3, 4, 5, 6, 7, 8, 9, 10, 11, 12, 13, 14, 15, 16 or 17 back-mutations selected from the residues 20, 34, 35, 38, 48, 50, 59, 61, 62, 70, 72, 74, 76, 77, 79, 82 and 95.

For more clarity, the following Table 4 illustrates the preferred back-mutations.

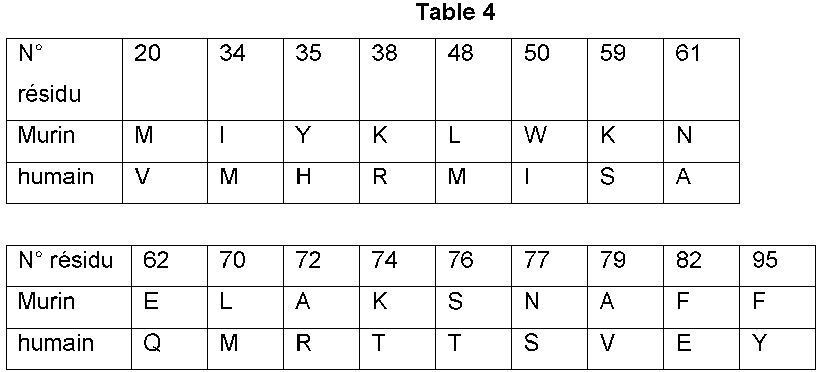

In an embodiment, the antibody comprises a light chain variable domain (VL) of sequence SEQ ID No. 35, wherein said sequence SEQ ID No. 35 comprises at least 1 back-mutation selected from the residues 22, 53, 55, 65, 71, 72, 77 and 87.

In an embodiment, the antibody comprises a light chain variable domain (VL) of sequence SEQ ID No. 35, wherein said sequence SEQ ID No. 35 comprises 2, 3, 4, 5, 6, 7 or 8 back-mutations selected from the residues 22, 53, 55, 65, 71, 72, 77 or 87.

In another embodiment, the antibody comprises:
a) a heavy chain variable domain (VH) of sequence SEQ ID No. 33 wherein said sequence SEQ ID No. 33 comprises at least 1 back-mutation selected from the residues 20, 34, 35, 38, 48, 50, 59, 61, 62, 70, 72, 74, 76, 77, 79, 82 and 95; and
b) a light chain variable domain (VL) of sequence SEQ ID No. 35, wherein said sequence SEQ ID No. 35 comprises at least 1 back-mutation selected from the residues 22, 53, 55, 65, 71, 72, 77 and 87.

For more clarity, the following Table 5 illustrates the preferred back-mutations.

**Table 5**

| N° résidu | 22 | 53 | 55 | 65 | 71 | 72 | 77 | 87 |
|---|---|---|---|---|---|---|---|---|
| Murin | S | R | H | R | Y | S | N | F |
| humain | T | S | Q | S | F | T | S | Y |

In such an embodiment, the antibody comprises all the back-mutations above mentioned and corresponds to an antibody comprising a heavy chain variable domain (VH) of sequence SEQ ID No. 34 and a light chain variable domain (VL) of sequence SEQ ID No. 36. Said humanized antibody will be called thereinafter hz208F2 ("Variant 3" or "Var. 3").

In another embodiment, all the humanized forms comprised between the Variant 1 and the Variant 3 are also encompassed by the present invention. In other words, the antibody corresponds to an antibody comprising a heavy chain variable domain (VH) of "consensus" sequence SEQ ID No. 41 and a light chain variable domain (VL) of "consensus" sequence SEQ ID No. 42. Said humanized antibody, as a whole, will be called thereinafter hz208F2 ("Variant2" or "Var.2").

In a particular embodiment, the antibody is selected from:
a) an antibody comprising a heavy chain variable domain of sequence SEQ ID No. 33 or any sequence exhibiting at least 80%, preferably 85%, 90%, 95% and 98% identity with SEQ ID No. 33 and the three light chain CDRs of sequences SEQ ID Nos. 9, 5 and 11; and
b) an antibody comprising a heavy chain variable domain of sequence SEQ ID No. 34 or any sequence exhibiting at least 80%, preferably 85%, 90%, 95% and 98% identity with SEQ ID No. 34 and the three light chain CDRs of sequences SEQ ID Nos. 9, 5 and 11.

By "any sequence exhibiting at least 80%, preferably 85%, 90%, 95% and 98% identity with SEQ ID No. 33 or 34", it is intended to designate the sequences exhibiting the three heavy chain CDRs SEQ ID Nos. 1, 2 and 3 and, in addition, exhibiting at least 80%, preferably 85%, 90%, 95% and 98%, identity with the full sequence SEQ ID No. 33 or 34 outside the sequences corresponding to the CDRs (i.e. SEQ ID Nos. 1, 2 and 3). In an embodiment of the invention, the antibody is selected from:
a) an antibody comprising a heavy chain variable domain of sequence SEQ ID No. 33 or any sequence exhibiting at least 80% identity with SEQ ID No. 33 and the three light chain CDRs of sequences SEQ ID Nos. 9, 5 and 11; and
b) an antibody comprising a heavy chain variable domain of sequence SEQ ID No. 34 or any sequence exhibiting at least 80% identity with SEQ ID No. 34 and the three light chain CDRs of sequences SEQ ID Nos. 9, 5 and 11.

If not indicated in the concerned paragraphs, in the present description, by any sequence or by a sequence exhibiting at least 80% with a particular sequence, it must be understood that said sequence exhibits at least 80% and preferably 85%, 90%, 95% and 98% identity with the referenced sequence. Whether these sequences contain CDR sequences, it is intended to designate that the sequences exhibiting at least these CDRs identically to the reference sequence CDRs, the 80%, preferably 85%, 90%, 95% and 98%, identity with the full sequence having to be calculated for the remaining sequence located outside the sequences corresponding to these CDRs.

In a particular embodiment, the antibody is selected from:
a) an antibody comprising a light chain variable domain of sequence SEQ ID No. 35 or any sequence exhibiting at least 80%, preferably 85%, 90%, 95% and 98% identity with SEQ ID No. 35 and the three heavy chain CDRs of sequences SEQ ID Nos. 7, 2 and 3; and
b) an antibody comprising a light chain variable domain of sequence SEQ ID No. 36 or any sequence exhibiting at least 80%, preferably 85%, 90%, 95% and 98% identity with SEQ ID No. 36 and the three heavy chain CDRs of sequences SEQ ID Nos. 7, 2 and 3.

By "any sequence exhibiting at least 80%, preferably 85%, 90%, 95% and 98% identity with SEQ ID No. 35 or 36", it is intended to designate the sequences exhibiting the three light chain CDRs SEQ ID Nos. 4, 5 and 6 and, in addition, exhibiting at least 80%, preferably 85%, 90%, 95% and 98%, identity with the full sequence SEQ ID No. 35 or 36 outside the sequences corresponding to the CDRs (i.e. SEQ ID Nos. 4, 5 and 6). In an embodiment of the invention, Ab is selected from:
a) an antibody comprising a light chain variable domain of sequence SEQ ID No. 35 or any sequence exhibiting at least 80% identity with SEQ ID No. 35 and the three heavy chain CDRs of sequences SEQ ID Nos. 7, 2 and 3; and
b) an antibody comprising a heavy chain variable domain of sequence SEQ ID No. 36 or any sequence exhibiting at least 80% identity with SEQ ID No. 36 and the three heavy chain CDRs of sequences SEQ ID Nos. 7, 2 and 3.

Humanized antibodies herein described can be also characterized by the constant domain and, more particularly, said humanized antibodies can be selected or designed such as, without limitation, IgG1, IgG2, IgG3, IgM, IgA, IgD or IgE. More preferably, in the context of the present invention, said humanized antibodies are IgG1 or IgG4.

An embodiment of the invention relates to an ADC wherein "Ab" is a humanized antibody comprising variable domains VH and VL as above described in the format IgG1. More preferably, said humanized antibody comprises a constant domain for the VH of sequence SEQ ID No. 43 and a Kappa domain for the VL of sequence SEQ ID No. 45.

An embodiment of the invention relates to an ADC wherein "Ab" is a humanized antibody comprising variable domains VH and VL as above described in the format IgG4. More preferably, said humanized antibody comprises a constant domain for the VH of sequence SEQ ID No. 44 and a Kappa domain for the VL of sequence SEQ ID No. 45. In still another embodiment, the antibody selected from:
a) an antibody comprising or consisting of a heavy chain of sequence SEQ ID No. 37 or any sequence exhibiting at least 80% identity with SEQ ID No. 37 and a light chain of sequence SEQ ID No. 39 or any sequence exhibiting at least 80% identity with SEQ ID No. 39; and
b) an antibody comprising or consisting of a heavy chain of sequence SEQ ID No. 38 or any sequence exhibiting at least 80% identity with SEQ ID No. 38 and a light chain of sequence SEQ ID No. 40 or any sequence exhibiting at least 80% identity with SEQ ID No. 40.

For more clarity, the following Tables 6a and 6b illustrate non limitative examples of sequences of the VH and VL for the variant 1 (Var. 1) and the variant 3 (Var. 3) of the humanized antibody hz208F2. It also comprises the consensus sequence for the variant 2 (Var. 2).

**Table 6a**

| | **Heavy Chain** | **Light chain** | **SEQ ID No.** |
|---|---|---|---|
| hz208F2 (var. 1) | Variable domain (VH) | | 33 |
| | | Variable domain (VL) | 35 |
| | Full length | | 37 |
| | | Full length | 39 |
| hz208F2 (Var. 3) | Variable domain (VH) | | 34 |
| | | Variable domain (VL) | 36 |
| | Full length | | 38 |
| | | Full length | 40 |
| hz208F2 (Var. 2) | Variable domain (VH) | | 41 |
| | | Variable domain (VL) | 42 |

**Table 6b**

| SEQ ID No. | Sequence |
|---|---|
| 33 | QVQLVQSGAE VKKPGASVKV SCKASGYTFT SYYMHWVRQA PGQGLEWMGI IWPGDGSTSY AQKFQGRVTM TRDTSTSTVY MELSSLRSED TAVYYCASPM ITPNYAMDYW GQGTLVTVSS |
| 34 | QVQLVQSGAE VKKPGASVKM SCKASGYTFT SYYIYWVKQA PGQGLEWLGW IWPGDGSTKY NEKFQGRVTL TADKSSNTAY MFLSSLRSED TAVYFCASPM ITPNYAMDYW GQGTLVTVSS |
| 35 | DIQMTQSPSS LSASVGDRVT ITCRASQDIS KYLNWYQQKP GKAPKLLIYY TSSLQSGVPS RFSGSGSGTD FTLTISSLQP EDFATYYCQQ GSTLPYTFGG GTKVEIK |
| 36 | DIQMTQSPSS LSASVGDRVT ISCRASQDIS KYLNWYQQKP GKAPKLLIYY TSRLHSGVPS RFSGRGSGTD YSLTISNLQP EDFATYFCQQ GSTLPYTFGG GTKVEIK |
| 37 | QVQLVQSGAE VKKPGASVKV SCKASGYTFT SYYMHWVRQA PGQGLEWMGI IWPGDGSTSY AQKFQGRVTM TRDTSTSTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 38 | QVQLVQSGAE VKKPGASVKM SCKASGYTFT SYYIYWVKQA PGQGLEWLGW IWPGDGSTKY NEKFQGRVTL TADKSSNTAY MFLSSLRSED TAVYFCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 39 | DIQMTQSPSS LSASVGDRVT ITCRASQDIS KYLNWYQQKP GKAPKLLIYY TSSLQSGVPS RFSGSGSGTD FTLTISSLQP EDFATYYCQQ GSTLPYTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 40 | DIQMTQSPSS LSASVGDRVT ISCRASQDIS KYLNWYQQKP GKAPKLLIYY TSRLHSGVPS RFSGRGSGTD YSLTISNLQP EDFATYFCQQ GSTLPYTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 41 | QVQLVQSGAE VKKPGASVKM SCKASGYTFT SYYIYWVKQA PGQGLEWLGW IWPGDGSTKY NEKFQGRVTL TADKSSNTAY MFLSSLRSED TAVYFCASPM ITPNYAMDYW GQGTLVTVSS (position 20: M may be replaced by V, position 34: I may be replaced by M; position 35: Y may be replaced by H; position 38: K may be replaced by R, position 48: L may be replaced by M, position 50: W may be replaced by I, position 59: K may be replaced by S; position 61: N may be replaced by A, position 62: E may be replaced by Q, position 70: L may be replaced by M, position 72: A may be replaced by R, position 74: K may be replaced by T, position 76: S may be replaced by T, position 77: N may be replaced by S, position 79: A may be replaced by V, position 82: F may be replaced by E, position 95: F may be replaced by Y) |

In another embodiment, the antibody is selected from:
a) an antibody comprising a heavy chain variable domain of sequence selected from SEQ ID Nos. 56, 62, 64, 66, 68, 70, 72, 74, 76, 78 and 54 or any sequence with at least 80%, preferably 85%, 90%, 95% and 98% identity with SEQ ID No.56, 62, 64, 66, 68, 70, 72, 74, 76, 78 and 54; and the three light chain CDRs of sequences SEQ ID Nos. 9, 5 and 11;
b) an antibody comprising a light chain variable domain of sequence selected from SEQ ID Nos. 57 or 60 or any sequence with at least 80%, preferably 85%, 90%, 95% and 98% identity with SEQ ID Nos. 57 or 60; and the three heavy chain CDRs of sequences SEQ ID Nos. 7, 2 and 3; and
c) an antibody comprising a heavy chain variable domain of sequence selected from SEQ ID Nos. 56, 62, 64, 66, 68, 70, 72, 74, 76, 78 and 54 or any sequence with at least 80%, preferably 85%, 90%, 95% and 98% identity with SEQ ID Nos.56, 62, 64, 66, 68, 70, 72, 74, 76, 78 and54; and a light chain variable domain of sequence selected from SEQ ID Nos. 57 or 60 or any sequence with at least 80%, preferably 85%, 90%, 95% and 98% identity with SEQ ID Nos. 57 or 60.

In still another embodiment, the antibody is selected from:
a) an antibody comprising a heavy chain of sequence SEQ ID Nos. 56, 62, 64, 66, 68, 70, 72, 74, 76, 78 and 54or any sequence exhibiting at least 80% identity with SEQ ID No. 56, 62, 64, 66, 68, 70, 72, 74, 76, 78 or 54, and a light chain of sequence SEQ ID No. 57 or any sequence exhibiting at least 80% identity with SEQ ID No. 57; and
b) an antibody comprising a heavy chain of sequence SEQ ID Nos. 56, 64, 68 and 78 or any sequence exhibiting at least 80% identity with SEQ ID No. 56, 64, 68 or 78 and a light chain of sequence SEQ ID No. 60, or any sequence exhibiting at least 80% identity with SEQ ID No. 60.

In still another embodiment, the antibody is selected from:
a) an antibody comprising or consisting of a heavy chain of sequence SEQ ID No. 58 or any sequence exhibiting at least 80% identity with SEQ ID No. 58 and a light chain of sequence SEQ ID No. 59 or any sequence exhibiting at least 80% identity with SEQ ID No. 59;
b) an antibody comprising or consisting of a heavy chain of sequence SEQ ID No. 58 or any sequence exhibiting at least 80% identity with SEQ ID No. 58 and a light chain of sequence SEQ ID No. 61 or any sequence exhibiting at least 80% identity with SEQ ID No. 61;
c) an antibody comprising or consisting of a heavy chain of sequence SEQ ID No. 63 or any sequence exhibiting at least 80% identity with SEQ ID No. 63 and a light chain of sequence SEQ ID No. 59 or any sequence exhibiting at least 80% identity with SEQ ID No. 59;
d) an antibody comprising or consisting of a heavy chain of sequence SEQ ID No. 65 or any sequence exhibiting at least 80% identity with SEQ ID No. 65 and a light chain of sequence SEQ ID No. 59 or any sequence exhibiting at least 80% identity with SEQ ID No. 59;
e) an antibody comprising or consisting of a heavy chain of sequence SEQ ID No. 65 or any sequence exhibiting at least 80% identity with SEQ ID No. 65 and a light chain of sequence SEQ ID No. 61 or any sequence exhibiting at least 80% identity with SEQ ID No. 61;
f) an antibody comprising or consisting of a heavy chain of sequence SEQ ID No. 67 or any sequence exhibiting at least 80% identity with SEQ ID No. 67 and a light chain of sequence SEQ ID No. 59 or any sequence exhibiting at least 80% identity with SEQ ID No. 59;
g) an antibody comprising or consisting of a heavy chain of sequence SEQ ID No. 69 or any sequence exhibiting at least 80% identity with SEQ ID No. 69 and a light chain of sequence SEQ ID No. 59 or any sequence exhibiting at least 80% identity with SEQ ID No. 59;
h) an antibody comprising or consisting of a heavy chain of sequence SEQ ID No. 69 or any sequence exhibiting at least 80% identity with SEQ ID No. 69 and a light chain of sequence SEQ ID No. 61 or any sequence exhibiting at least 80% identity with SEQ ID No. 61;
i) an antibody comprising or consisting of a heavy chain of sequence SEQ ID No. 71 or any sequence exhibiting at least 80% identity with SEQ ID No. 71 and a light chain of sequence SEQ ID No. 59 or any sequence exhibiting at least 80% identity with SEQ ID No. 59;
j) an antibody comprising or consisting of a heavy chain of sequence SEQ ID No. 73 or any sequence exhibiting at least 80% identity with SEQ ID No. 73 and a light chain of sequence SEQ ID No. 59 or any sequence exhibiting at least 80% identity with SEQ ID No. 59;
k) an antibody comprising or consisting of a heavy chain of sequence SEQ ID No. 75 or any sequence exhibiting at least 80% identity with SEQ ID No. 75 and a light chain of sequence SEQ ID No. 59 or any sequence exhibiting at least 80% identity with SEQ ID No. 59;
l) an antibody comprising or consisting of a heavy chain of sequence SEQ ID No. 77 or any sequence exhibiting at least 80% identity with SEQ ID No. 77 and a light chain of sequence SEQ ID No. 59 or any sequence exhibiting at least 80% identity with SEQ ID No. 59;
m) an antibody comprising or consisting of a heavy chain of sequence SEQ ID No. 53 or any sequence exhibiting at least 80% identity with SEQ ID No. 53 and a light chain of sequence SEQ ID No. 59 or any sequence exhibiting at least 80% identity with SEQ ID No. 59;
n) an antibody comprising or consisting of a heavy chain of sequence SEQ ID No. 53 or any sequence exhibiting at least 80% identity with SEQ ID No. 53 and a light chain of sequence SEQ ID No. 61 or any sequence exhibiting at least 80% identity with SEQ ID No. 61; and
o) an antibody comprising or consisting of a heavy chain of sequence SEQ ID No. 55 or any sequence exhibiting at least 80% identity with SEQ ID No. 55 and a light chain of sequence SEQ ID No. 59 or any sequence exhibiting at least 80% identity with SEQ ID No. 59.

In other words, the antibody can comprise:
a) a heavy chain of sequence selected from SEQ ID Nos. 58, 63, 65, 67, 69, 71, 73, 75, 77, 53 and 55 or any sequence with at least 80% identity with SEQ ID Nos. 58, 63, 65, 67, 69, 71, 73, 75, 77, 53 and 55; and
b) a light chain of sequence selected from SEQ ID Nos. 59 and 61 or any sequence with at least 80% identity with SEQ ID Nos. 59 and 61.

For more clarity, the following Tables 6c and 6d illustrate non limitative examples of sequences of the VH and VL (variable domain and full length) for different variants of the humanized antibody hz208F2.

**Table 6c**

| | **Heavy Chain** | **Light chain** | **SEQ ID NO.** |
|---|---|---|---|
| | Variable domain (VH) | | 56 |
| hz208F2 | | Variable domain (VL) | 57 |
| H037/L018 | Full length | | 58 |
| | | Full length | 59 |
| | Variable domain (VH) | | 56 |
| Hz208F2 | | Variable domain (VL) | 60 |
| H037/L021 | Full length | | 58 |
| | | Full length | 61 |
| | Variable domain (VH) | | 62 |
| Hz208F2 | | Variable domain (VL) | 57 |
| H047/L018 | Full length | | 63 |
| | | Full length | 59 |
| | Variable domain (VH) | | 64 |
| Hz208F2 | | Variable domain (VL) | 57 |
| H049/L018 | Full length | | 65 |
| | | Full length | 59 |
| | Variable domain (VH) | | 64 |
| Hz208F2 | | Variable domain (VL) | 60 |
| H049/L021 | Full length | | 65 |
| | | Full length | 61 |
| | Variable domain (VH) | | 66 |
| Hz208F2 | | Variable domain (VL) | 57 |
| H051/L018 | Full length | | 67 |
| | | Full length | 59 |
| | Variable domain (VH) | | 68 |
| Hz208F2 | | Variable domain (VL) | 57 |
| H052/L018 | Full length | | 69 |
| | | Full length | 59 |
| | Variable domain (VH) | | 68 |
| Hz208F2 | | Variable domain (VL) | 60 |
| H052/L021 | Full length | | 69 |
| | | Full length | 61 |
| | Variable domain (VH) | | 70 |
| Hz208F2 | | Variable domain (VL) | 57 |
| H057/L018 | Full length | | 71 |
| | | Full length | 59 |
| | Variable domain (VH) | | 72 |
| Hz208F2 | | Variable domain (VL) | 57 |
| H068/L018 | Full length | | 73 |
| | | Full length | 59 |
| | Variable domain (VH) | | 74 |
| Hz208F2 | | Variable domain (VL) | 57 |
| H070/L018 | Full length | | 75 |
| | | Full length | 59 |
| | Variable domain (VH) | | 76 |
| Hz208F2 | | Variable domain (VL) | 57 |
| H071/L018 | Full length | | 77 |
| | | Full length | 59 |
| | Variable domain (VH) | | 78 |
| Hz208F2 | | Variable domain (VL) | 57 |
| H076/L018 | Full length | | 53 |
| | | Full length | 59 |
| | Variable domain (VH) | | 78 |
| Hz208F2 | | Variable domain (VL) | 60 |
| H076/L021 | Full length | | 53 |
| | | Full length | 61 |
| | Variable domain (VH) | | 54 |
| Hz208F2 | | Variable domain (VL) | 57 |
| H077/L018 | Full length | | 55 |
| | | Full length | 59 |

**Table 6d**

| SEQ ID No. | Sequence |
|---|---|
| 53 | QVQLVQSGAE VKKPGASVKV SCKASGYTFT SYYIHWVRQA PGQGLEWMGW IWPGDGSTKY AQKFQGRVTM TRDTSTSTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 54 | QVQLVQSGAE VKKPGASVKV SCKASGYTFT SYYIHWVRQA PGQGLEWMGW IWPGDGSTKY AQKFQGRVTM TRDTSTSTVY MELSSLRSED TAVYFCASPM ITPNYAMDYW GQGTLVTVSS |
| 55 | QVQLVQSGAE VKKPGASVKV SCKASGYTFT SYYIHWVRQA PGQGLEWMGW IWPGDGSTKY AQKFQGRVTM TRDTSTSTVY MELSSLRSED TAVYFCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 56 | QVQLVQSGAE VKKPGASVKV SCKASGYTFT SYYMHWVRQA PGQGLEWMGW IWPGDGSTKY AQKFQGRVTM TRDKSSSTVY MELSSLRSED TAVYFCASPM ITPNYAMDYW GQGTLVTVSS |
| 57 | DIQMTQSPSS LSASVGDRVT ITCRASQDIS KYLNWYQQKP GKAPKLLIYY TSRLQSGVPS RFSGRGSGTD YSLTISSLQP EDFATYFCQQ GSTLPYTFGG GTKVEIK |
| 58 | QVQLVQSGAE VKKPGASVKV SCKASGYTFT SYYMHWVRQA PGQGLEWMGW IWPGDGSTKY AQKFQGRVTM TRDKSSSTVY MELSSLRSED TAVYFCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 59 | DIQMTQSPSS LSASVGDRVT ITCRASQDIS KYLNWYQQKP GKAPKLLIYY TSRLQSGVPS RFSGRGSGTD YSLTISSLQP EDFATYFCQQ GSTLPYTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 60 | DIQMTQSPSS LSASVGDRVT ITCRASQDIS KYLNWYQQKP GKAPKLLIYY TSRLHSGVPS RFSGRGSGTD FSLTISSLQP EDFATYFCQQ GSTLPYTFGG GTKVEIK |
| 61 | DIQMTQSPSS LSASVGDRVT ITCRASQDIS KYLNWYQQKP GKAPKLLIYY TSRLHSGVPS RFSGRGSGTD FSLTISSLQP EDFATYFCQQ GSTLPYTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 62 | QVQLVQSGAE VKKPGASVKV SCKASGYTFT SYYIHWVRQA PGQGLEWMGW IWPGDGSTKY AQKFQGRVTM TRDTSSNTVY MELSSLRSED TAVYYCASPM ITPNYAMDYW GQGTLVTVSS |
| 63 | QVQLVQSGAE VKKPGASVKV SCKASGYTFT SYYIHWVRQA PGQGLEWMGW IWPGDGSTKY AQKFQGRVTM TRDTSSNTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 64 | QVQLVQSGAE VKKPGASVKV SCKASGYTFT SYYMHWVRQA PGQGLEWMGW IWPGDGSTKY AQKFQGRVTM TRDKSSSTVY MELSSLRSED TAVYYCASPM ITPNYAMDYW GQGTLVTVSS |
| 65 | QVQLVQSGAE VKKPGASVKV SCKASGYTFT SYYMHWVRQA PGQGLEWMGW IWPGDGSTKY AQKFQGRVTM TRDKSSSTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 66 | QVQLVQSGAE VKKPGASVKV SCKASGYTFT SYYMHWVRQA PGQGLEWMGW IWPGDGSTKY AQKFQGRVTM TRDTSSNTVY MELSSLRSED TAVYFCASPM ITPNYAMDYW GQGTLVTVSS |
| 67 | QVQLVQSGAE VKKPGASVKV SCKASGYTFT SYYMHWVRQA PGQGLEWMGW IWPGDGSTKY AQKFQGRVTM TRDTSSNTVY MELSSLRSED TAVYFCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 68 | QVQLVQSGAE VKKPGASVKV SCKASGYTFT SYYMHWVRQA PGQGLEWMGW IWPGDGSTKY AQKFQGRVTM TRDTSTSTVY MELSSLRSED TAVYFCASPM ITPNYAMDYW GQGTLVTVSS |
| 69 | QVQLVQSGAE VKKPGASVKV SCKASGYTFT SYYMHWVRQA PGQGLEWMGW IWPGDGSTKY AQKFQGRVTM TRDTSTSTVY MELSSLRSED TAVYFCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 70 | QVQLVQSGAE VKKPGASVKV SCKASGYTFT SYYIHWVRQA PGQGLEWMGW IWPGDGSTKY AQKFQGRVTM TRDKSTNTVY MELSSLRSED TAVYYCASPM ITPNYAMDYW GQGTLVTVSS |
| 71 | QVQLVQSGAE VKKPGASVKV SCKASGYTFT SYYIHWVRQA PGQGLEWMGW IWPGDGSTKY AQKFQGRVTM TRDKSTNTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 72 | QVQLVQSGAE VKKPGASVKV SCKASGYTFT SYYMHWVRQA PGQGLEWMGW IWPGDGSTKY AQKFQGRVTM TRDTSTNTVY MELSSLRSED TAVYFCASPM ITPNYAMDYW GQGTLVTVSS |
| 73 | QVQLVQSGAE VKKPGASVKV SCKASGYTFT SYYMHWVRQA PGQGLEWMGW IWPGDGSTKY AQKFQGRVTM TRDTSTNTVY MELSSLRSED TAVYFCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 74 | QVQLVQSGAE VKKPGASVKV SCKASGYTFT SYYMHWVRQA PGQGLEWMGW IWPGDGSTKY AQKFQGRVTM TRDTSTSTVY MELSSLRSED TAVYYCASPM ITPNYAMDYW GQGTLVTVSS |
| 75 | QVQLVQSGAE VKKPGASVKV SCKASGYTFT SYYMHWVRQA PGQGLEWMGW IWPGDGSTKY AQKFQGRVTM TRDTSTSTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 76 | QVQLVQSGAE VKKPGASVKV SCKASGYTFT SYYMHWVRQA PGQGLEWMGW IWPGDGSTKY AQKFQGRVTM TRDTSTNTVY MELSSLRSED TAVYYCASPM ITPNYAMDYW GQGTLVTVSS |
| 77 | QVQLVQSGAE VKKPGASVKV SCKASGYTFT SYYMHWVRQA PGQGLEWMGW IWPGDGSTKY AQKFQGRVTM TRDTSTNTVY MELSSLRSED TAVYYCASPM ITPNYAMDYW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKRVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPG |
| 78 | QVQLVQSGAE VKKPGASVKV SCKASGYTFT SYYIHWVRQA PGQGLEWMGW IWPGDGSTKY AQKFQGRVTM TRDTSTSTVY MELSSLRSED TAVYYCASPM ITPNYAMDYW GQGTLVTVSS |

According to another aspect of the present invention, the antibody is produced by the hybridoma I-4757, I-4773, I-4775, I-4736 or I-4774 deposited at the CNCM, Institut Pasteur France on the 30 May 2013, 26 June 2013, 26 June 2013, 24 April 2013 and 26 June 2013 (see WO2015162292).

The antibody is more particularly a 208F2 antibody (e.g. chimeric or humanized, especially chimeric) and more particularly a c208F2 antibody which is a monoclonal antibody (IgG1). This antibody is notably described in WO2015162291 and is characterized in that it comprises the three light chain CDRs of sequence SEQ ID Nos. 9, 5 and 11 respectively, and the three heavy chain CDRs of sequence SEQ ID Nos. 7, 2 and 3 respectively. The antibody is further characterized in that it comprises a light chain variable domain of sequence SEQ ID No. 18 and a heavy chain variable domain of sequence SEQ ID No. 13. The antibody is finally characterized in that it comprises a light chain variable domain of sequence SEQ ID No 28 and a heavy chain variable domain of sequence SEQ ID No 23.

The c208F2 antibody and the ADC of formula (V) can be obtained by all the methods known to the man of the art for obtaining antibodies and ADCs. In particular, they are obtained by the methods described in WO2015162291.

### Use or method according to the invention

The liquid aqueous composition according to the invention is used for solubilization and stabilization of an antibody-drug conjugate (ADC) during a period T equal or greater than 3 months at a temperature Θ of from 2°C to 8°C.

According to a first embodiment, the stabilization of the ADC is defined as a percentage of aggregates in the composition resulting from the solubilization of the ADC in the liquid aqueous composition according to the invention of less than 5% of aggregates. The percentage of aggregates can be measured by size exclusion chromatography, in particular according to method 1 or 2 as described in the examples (see "Size variants by Size Exclusion Chromatography (SEC-UV)").

According to a second embodiment, the stabilization of the ADC is defined as a percentage of monomers in the composition resulting from the solubilization of the ADC in the liquid aqueous composition according to the invention of at least 90% of monomers. The percentage of monomers can be measured by size exclusion chromatography, according to method 1 or 2 as described in the examples (see "Size variants by Size Exclusion Chromatography (SEC-UV)").

According to a third embodiment, the stabilization of the ADC is defined as a percentage of free drug in the composition resulting from the solubilization of the ADC in the liquid aqueous composition according to the invention equal or less than 0.1% of antibody-drug conjugate. The percentage of free drug can be measured by liquid chromatography (LC), in particular according to the method reported in the examples (see "Residual small molecular drugs (SMDs) and related substances").

According to a preferred embodiment, the stabilization of the ADC is defined as a percentage of aggregates of less than 5% of aggregates and a percentage of monomers of at least 90% of monomers.

According to a most preferred embodiment, the stabilization of the ADC is defined as a percentage of aggregates of less than 5% of aggregates, a percentage of monomers of at least 90% of monomers, and a percentage of aggregates of less than 5% of aggregates and a percentage of monomers of at least 90% of monomers.

According to a particular embodiment, the period T is equal or greater than 24 months, preferably equal or greater than 36 months, and preferably equal or greater than 48 months.

According to another embodiment, the period T is from 12 to 48 months.

### EXAMPLES:

### Example 1 - Stability after 0.5 to 2 months of storage

The following aqueous formulations were tested:
**1:** 25 mM Histidine pH 6.5, 150 mM NaCl, 6% Sucrose, 0.005% Polysorbate 80,
**2:** 25 mM Histidine pH 6.5, 150 mM NaCl, 6% Sucrose, 0.05% Polysorbate 80, and
**3:** 25 mM Histidine pH 6.5, 150 mM NaCl, 0.05% Polysorbate 80.

The ADC used was ADC **A** (corresponding to the ADC named hz208F2-G-13 in WO2015162291) at 5 mg/mL of aqueous formulation.

A stability study was performed with storage at -66°C, +5°C, +25°C and +40°C for 0.5, 1, 1.5 and 2 months. The following tests were performed at each time point: appearance, opalescence, pH, SEC-UV, DSF, HIC-HPLC, LC-MS (DAR and free drug), hIGF-1R binding (ELISA) and *in vitro* cytotoxicity.

ADCs were characterized by SEC-UV to evaluate the presence of aggregates, HIC-HPLC to calculate the mean DAR (and the percentage of odd DAR) and differential scanning fluorimetry (DSF) to determine the melting temperature (Tm).

There was no statistical effect of the different formulations on the binding capacity and the *in vitro* cytotoxicity of the ADC after 2 months storage, whatever the temperature.

The major differences were more specifically observed on the aggregates, the DAR, the Tm and on the released drug after storage at +40°C, which were unfavourable to the formulations 2 and 3.

### Example 2 - Stability after 1 to 48 months of storage

### 1. Description and composition of the tested drug product

The drug product comprising ADC A is a solution for infusion. It is clear to slightly opalescent, colourless to pale yellow solution.

It is supplied as a sterile solution intended for infusion after introduction in a sterile pouch of NaCl 0.9% solution. The concentration in protein of the drug product is 5 mg/mL.

The qualitative and quantitative composition of the drug product is as follows (for a 10 mL vial):

| | |
|---|---|
| - ADC A | 50.0 mg |
| - L Histidine (Ph. Eur. 0911) | 38.8 mg |
| - Sodium chloride (Ph. Eur. 0193) | 88 mg |
| - Sucrose (Ph. Eur. 0204) | 600 mg |
| - Polysorbate 80 (Ph. Eur. 0428) | 0.5 mg |
| - HCI 25% | qs pH 6.5 |
| - Water for injection (Ph. Eur. 0169) | qs to 10 mL |

The various batches testes are presented in Table 7 below.

**Table 7: Batches of the tested drug product**

| **Batch number** | **Batch size (unit)** | **Strength** | **Vial volume** |
|---|---|---|---|
| 1 | 628 | 5 mg/mL | 10 mL vial filled with 5 mL solution |
| 2 | 608 | 5 mg/mL | 13.5 mL vial filled with 10 mL solution |
| 3 | 565 | 5 mg/mL | 13.5 mL vial filled with 10.5 mL solution |
| 4 | 591 | 5 mg/mL | 13.5 mL vial filled with 10.5 mL solution |
| 5 | 716 | 5 mg/mL | 13.5 mL vial filled with 10.5 mL solution |
| **Packaging:** Type I colourless glass vial, 20 mm bromobutyl rubber stopper coated with a fluororesin film and 20 mm flip-off aluminum seal cap | | | |

| | | | |
|---|---|---|---|
| * overfill of 5% so as to allow the withdrawal of 10 mL of solution containing 50 mg of ADC A. | | | |

### 2. Characterisation of ADC A at T0

### Sub-visible particles

Aggregation is one of the main ways of protein degradation. Protein aggregates formation exists at all steps of pharmaceutical manufacturing. Aggregates may be of concern because they have the potential to trigger immune response that may impact safety and efficacy of the product.

Sub-visible particles may be the hallmark of aggregation or may be of foreign origin (fibers, silicone droplets). The ADC was checked for sub-visible particles using the European Pharmacopoeia methods 2.9.19 (Light obscuration and Microscopy) for quantification and the MicroFlow Imaging (MFI) technique for quantification and characterization.

According to the European Pharmacopoeia for the determination of particulate contamination, two procedures, Method 1 (Light Obscuration Particle Count Test) and Method 2 (Microscopic Particle Count Test), are specified.

Table 8 shows the result obtained for drug product batches 1 and 2.

**Table 8: Sub-visible particles in batches 1 and**

| Batch | Batch 1 | | | Batch 2 | | |
|---|---|---|---|---|---|---|
| Method | Light Obscuration⁽¹⁾ (Ph. Eur. 2.9.19 Method 1) | | MFI⁽³⁾ | Microscopy⁽²⁾ (Ph. Eur. 2.9.19 Method 2) | | MFI⁽³⁾ |
| | per mL | per vial (5 mL) | per mL | per mL | per vial (10 mL) | per mL |
| < 2 µm | ND | ND | 25,170 | ND | ND | 23,758 |
| ≥ 2 and < 5 µm | ND | ND | 8,192 | ND | ND | 4,332 |
| ≥ 5 and < 10 µm | ND | ND | 1,728 | ND | ND | 711 |
| ≥ 10 µm | 78 | 391 | 516 | < 1 | 9 | 308 |
| ≥ 25 µm | 1 | 5 | 46 | < 1 | 2 | 3 |

| | | | | | | |
|---|---|---|---|---|---|---|
| ⁽¹⁾ Light obscuration specifications: ≤6000 particles/vial for ≥ 10 µm and ≤600 particles/vial for ≥ 25 µm ⁽²⁾ Microscopy specifications: ≤3000 particles/vial for ≥ 10 µm and ≤300 particles/vial for ≥ 25 µm ⁽³⁾ No specifications are set for MFI ND: Not Done | | | | | | |

For particles of size ≥ 10 µm and ≥ 25 µm, counts for individual vials are in compliance with specifications for solutions for infusion.

Absolute counts for ≥ 10 µm and ≥ 25 µm with Micro-Flow Imaging (MFI) technique were slightly higher than light obscuration and microscopy quantifications. This is expected with MFI which detects 5-10 times more sub-visible particles in the >10µm range than the light obscuration method (Huang, J. Pharm Sci, 98, 2009; Sharma, AAPS Journal, 12, 2010). The images gathered by MFI showed a mixture of spherical particles (putatively air bubbles) and particles with heterogeneous shapes that tend to be more prevalent as size increases. Those were attributed to protein particles. Although the absolute number of ≥ 10 µm and ≥ 25 µm particles are within specifications, the formulation of ADC is 0.2µm in-line filtered during infusion to ensure additional patient protection.

### Elemental impurities

Elemental impurities were analyzed in batch 1 by ICP-MS according to Ph. Eur. 2.2.58 (23 elemental impurities + Al and Os). Results are reported in Table 9.

**Table 9: Elemental impurities**

| Batch number | Batch 1 |
|---|---|
| 25 elemental impurities | All elements < LOQ (from 0.005 to 0.75 µg/g) |

### Justification of specifications

**Table 10: Justification of ADC A batch specifications**

| **Tests** | **Acceptance criteria** | **Justification** |
|---|---|---|
| Appearance | Clear to slightly opalescent, colourless to pale yellow solution | The appearance criteria are in accordance with the characteristic generally described for biological solutions. |
| Identity by hydrophobic interaction | Complies with reference | Compendial guidelines (ICH Q6B, EMEA/CHMP/BWP/157653/2007) state that the identity test(s) should be highly specific and should be based on unique aspects of the product's molecular structure and/or other specific properties. HIC profile and peak intensities represent a characteristic signature of the ADC. Binding of the ADC to the IGF-1R is also a key identification element. |
| Identity by IGF-1R binding (ELISA) | Positive | |
| Fragment profile by LC | Complies with reference | Similar to published profiles for hinge-cys drug conjugates such as brentuximab vedotin |
| pH | 6.0 to 7.0 | Target pH for the product is 6.5. pH range within 0.5 unit of the target is expected for a buffered solution |
| Average DAR by HIC | 3.5 to 4.5 | Similar to published profiles for hinge-cys drug conjugates such as brentuximab vedotin |
| IGF-1R binding by ELISA | 50 to 150 % of standard reference | Compendial guidelines (ICH Q6B, EMEA/CHMP/BWP/157653/2007) state that relevant potency should be part of the specification for a biotechnological substance. Binding to IGF-1R receptor is the primary part of the ADC biological activity. |
| Biological activity (cytotoxicity) | IC50 ratio (in % of reference substance) 50 to 200 | Compendial guidelines (ICH Q6B, EMEA/CHMP/BWP/157653/2007) state that relevant potency should be part of the specification for a biotechnological substance. Cytotoxicity assay is relevant to quantify the ADC activity. |
| Size variants by size exclusion chromatography | IgG Monomer: ≥ 90 % IgG aggregates: ≤ 5% | For monomer content acceptance criteria is based on a recent International Consortium for Innovation and Quality in Pharmaceutical Development white paper⁽¹⁾ Aggregation is considered critical for product quality and clinical safety. For aggregates the acceptance criteria was set at not more than 5% in accordance with limits generally proposed for therapeutic biologics. |
| Drug loading and distribution by HIC | D0 ≤10% | Similar to published profiles for hinge-cys drug conjugates such as brentuximab vedotin |
| Residual small molecular drug and related substances by LC (Sum % w/w (drug/ADC)) | ≤ 0.1% | The quantity of residual drug/linker (i.e. drug, linker and drug-linker conjugate) should be determined in case of ADC drug product. The acceptance criteria stated is generally used for ADC drug products. |
| Residual DMSO by LC (ppm) | ≤ 5000 | ICH Q3C specification for DMSO (class 3 solvent) |
| Bacterial endotoxins | < 0.5 IU/mg of protein | The level set for endotoxin is in accordance with the Ph Eur criteria defined for parenteral administration of 5UI/kg of corporal mass for the maximal dose administrated in 1 hour. A maximal ADC dose of 10mg/kg was taken into account for setting the specification. |
| Microbiological examination | TAMC < 1 CFU/10 mL TYMC < 1 CFU/10 mL | Bioburden determination must be performed before sterile-filtration. A very low level of contamination is required for the drug substance bulk. |
| Protein content | 4.5 to 5.5 mg/mL | The quantity of the drug substance, usually based on protein content (mass), should be determined using an appropriate assay (EMEA/CHMP/BWP/157653/2007). The release specification is intended to ensure adequate and accurate dosage of the product. |

| | | |
|---|---|---|
| ⁽¹⁾ Kretsinger J. et al. J Pharm Sci. 2019, 108(4):1442-1452. doi: 10.1016/j.xphs.2018.11.042 | | |

### 3. Stability

### Protocol

The stability testing protocol is presented in Table 11.

**Table 11: Stability testing protocol applied on drug product batches 1 to 5 in long-term conditions (5±3°C)**

| **Methods** | **Test intervals (months)** | | | | | | | |
|---|---|---|---|---|---|---|---|---|
| | **T0** | **6** | **9** | **12** | **18** | **24** | **36** | **48** |
| Appearance | X | X | X | X | X | X | X | ) |
| Identification : fragment profile by LC | X | X | X | X | X | X | X | X |
| Degree of opalescence | X | X | X | X | X | X | X | X |
| pH | X | X | X | X | X | X | X | X |
| Average DAR by HIC | X | X | X | X | X | X | X | X |
| Target binding by ELISA - EC50 ratio (potency) | X | X | X | X | X | X | X | X |
| Biological activity (cytotoxicity) - IC50 ratio | X | X | X | X | X | X | X | X |
| Size variants by SEC-UV | X | X | X | X | X | X | X | X |
| Drug loading and distribution by HIC | X | X | X | X | X | X | X | X |
| Residual drug and related substances by LC | X | X | X | X | X | X | X | X |
| Particulate contamination: sub-visible particles (Ph. Eur 2.9.19. and/or by MFI) | X | X | - | X | - | X | X | X |
| Total charge variants by icIEF | X | X | X | X | X | X | X | X |
| Bacterial endotoxins | X | X | - | X | - | X | X | X |
| Protein content by UV | X | X | X | X | X | X | X | X |
| Protein content by HPLC protein A | X | X | X | X | X | X | X | X |
| Sterility | X | X | - | X | - | X | X | X |

### Methods

*Average DAR and drug loading and distribution* by *HIC*

Hydrophobic interaction chromatography (HIC) separates molecules based on their hydrophobicity in their native state. HIC is used to determinate the drug load distribution and the average DAR of the ADC.

Average drug-to-antibody ratio (DAR) is assessed according to the LC method (HIC) described below. The sample is diluted 1/2 in mobile phase A and analysed as described in the table :

| | | | |
|---|---|---|---|
| Detector | UV detector | | |
| | λ = 280 nm | | |
| Column | TSK-gel butyl-NPR : (Tosoh bioscience) | | |
| | 28°C | | |
| Mobile phase | A : 25 mM potassium phosphate, 1.5M ammonium sulfate, pH = 7.0 | | |
| | B : 25 mM potassium phosphate, 25% isopropanol | | |
| | **Time (min)** | **%A** | **%B** |
| Gradient | 0 | 90 | 10 |
| | 1.6-t^{∗} | 90 | 10 |
| | 13.6-t | 0 | 100 |
| ^{∗}Dwell time of system | 15.1-t | 0 | 100 |
| | 19.1-t | 90 | 10 |
| | 26.1-t | 90 | 10 |
| Flow rate | 0.8 mL/min | | |
| Injection volume | 5 to 20 µL | | |

### Total charge variants by icIEF

Imaged Capillary isoelectric focusing (icIEF) was used to determine ADC isoelectric point (pl) of main peak and evaluate ADC sample heterogeneity (giving different isoelectric points). icIEF separates proteins based on their isoelectric point (pl).

Measurements were carried out using an iCE3 clEF analyser system equipped with a UV detector at 280 nm. The catholyte is 0.1 M NaOH in 0.1% methyl cellulose and the anolyte is 0.08 M phosphoric acid in 0.1% methyl cellulose. The sample is diluted to 0.3 mg/mL in a solution containing isoelectric focusing markers.

The quantification is achieved by normalization procedure. Results for acidic variants, main peak and basic variants are reported as relative % of total area.

### Fragment profile by LC

The ADC is reduced into two chains (light chain and heavy chain) which may carry payloads: up to one for the light chain and up to three for the heavy chain and which are designed respectively as L0, L1, H0, H1, H2 & H3.

These ADC fragments are adsorbed on the reversed phase column thanks to hydrophobic interactions. They are then eluted by increasing the amount of eluting solution, i.e. acetonitrile + 0.05% TFA (versus MilliQ water + 0.05% TFA), during the chromatographic gradient.

UV detection at 210 nm of the separated ADC fragments results in the drug load profile which is used for the average DAR calculation. MS detection is achieved in parallel to check the identity of each fragment. The mass spectrometer consists in an electrospray (ESI) source and a time-of-flight (TOF) analyzer.

### Potency and biological activity

### Target binding by IGF-1R (Elisa) - EC50 ratio

The potency of ADC A is studied by IGF-1F binding performed by ELISA.

This test assesses direct binding between ADC A and IGF-1R receptor coated onto a 96-well plate. After plate blocking, ADC is serially diluted across the plate to allow binding with receptors. The complex is detected with goat anti-human IgG, conjugated to Horseradish Peroxydase (HRP) and quantified using a colorimetric substrate. Four-parameter logistic fits are used for data analysis to derive EC50 values for ADC A which is compared to the reference EC50 value.

### Size variants by Size Exclusion Chromatography (SEC-UV)

Size exclusion chromatography is used to study the average size variants of the drug product in native conditions.

A method 1 based on Liquid Chromatography (Ph. Eur. 2.2.29) was developed according to the conditions below (Table 12). The sample is diluted at 1.5 mg/mL in mobile phase. The quantification is achieved by normalization procedure. Results for aggregates, monomer and fragments peaks are reported as relative % of total area.

**Table 12: Chromatography conditions for size variants method 1**

| | |
|---|---|
| Detector | UV detector |
| | λ=280 nm |
| Column | TSKgel G3000 SW_{XL} (Tosoh Bioscience) 300 × 7.8 mm, 300Å and TSKgel SWXL (Tosoh Bioscience) 40 × 6.0 mm |
| | Temperature: 30°C |
| Mobile phase | A: 25 mM Na₂HPO₄/500 mM NaCl, pH7.0 |
| Isocratic mode | Run: 35 min |
| Flow rate | 0.5 mL/min |
| Injection volume | 15 µL |
| Injector | 15°C |

A method 2 has been developed including a different column and reduced salt concentration in mobile phase (see conditions in Table 13).

The sample is diluted at 3.0 mg/mL in purified water. The quantification is achieved by normalization procedure. Results for aggregates, monomer and fragments peaks are reported as relative % of total area.

**Table 13: Chromatography conditions for size variants method 2**

| | |
|---|---|
| Detector | UV detector λ=280 nm |
| Column | MabPac SEC-1 5µm 300A 7,8 × 300mm (Thermo Scientific) |
| | Temperature: 30°C |
| Mobile phase | A: 50 mM sodium phosphate buffer/300 mM NaCl, pH6.8 |
| Isocratic mode | Run: 35 min |
| Flow rate | 0.5 mL/min |
| Injection volume | 20 µL |
| Injector | 4°C |

### Size variants by Asymmetrical Flow Field-Flow Fractionation (A4F)

Asymmetrical Flow Field-Flow Fractionation is an analytical technology that separates protein by size through flow retention from few nanometer to few micrometer in diameter. Evaluation of size variants was monitored by A4F with UV absorption at 280 nm and multiangle laser light scattering detection (MALLS).

Samples are injected as is into an A4F separation system (Wyatt Eclipse Dualtec) equipped with a 10 kDa porosity cellulose membrane. The integrated 280 nm UV signal gives the relative amounts of monomeric, dimeric, aggregates and fragments protein, while signal MALLS detector allows calculating molecular mass of eluted compounds.

### Residual small molecular drugs (SMDs) and related substances by liquid chromatography (LC)

ADC, residual small molecular drug and its impurities are eluted at different retention times, due to their hydrophobicity difference and a specific 450 Å chromatographic column. The ADC has more interactions with stationary phase and can be easily separated from the different residual drugs.

Residual SMDs and related substances are assessed according to the LC method described below. An amount of exactly 250µg of drug product is injected. The sum of residual drug peaks in ADC product is compared to the residual drug reference injected at 1000 ppm corresponding to 0.1% w/w. The ADC product is analysed as described in Table 14.

**Table 14: Chromatographic conditions for analysis of residual SMDs and related substances**

| | | | |
|---|---|---|---|
| Detector | UV detector | | |
| | A = 210 nm | | |
| Column | AdvanceBio RP-mAB C4 100mm × 2.1mm (450Å) (Agilent) | | |
| | 40°C | | |
| Mobile phase | A: 0.05%TFA in water purified | | |
| | B: 0.05% TFA in acetonitrile | | |
| Gradient | **Time (min)** | **%A** | **%B** |
| | 0 | 90 | 10 |
| | 1 | 90 | 10 |
| | 12 | 68 | 32 |
| | 16.5 | 68 | 32 |
| | 17 | 10 | 90 |
| | 25 | 10 | 90 |
| | 26 | 0 | 100 |
| | 30 | 0 | 100 |
| | 31 | 90 | 10 |
| | 41 | 90 | 10 |
| Flow rate | 0.3 mL/min | | |
| Injection volume | 50 µL | | |

### Protein content by UV spectrophotometry

The protein concentration in drug product is determined by UV spectrophotometry at 280 nm. Sample is diluted with purified water to give an absorbance reading in the working range of 0.3 to 1.0 AU. The protein concentration is calculated dividing the absorbance reading by the determined ADC, 280 nm specific absorbance (1.448 AU.mg.mL⁻¹.cm⁻¹). The value is then corrected of the diluted factor.

### Results

After **18 months** of storage and for all the batches in long term storage conditions (5°C ± 3°C), the results are within the acceptance criteria and no significant change of the ADC profile is pointed out.

After **24 months** of storage and for all the tested batches (i.e. batches 1-4) in long term storage conditions (5°C ± 3°C), the results are within the acceptance criteria and no significant change of the ADC profile is pointed out.

After **36 months** of storage and for all the tested batches (i.e. batches 1-3) in long term storage conditions (5°C ± 3°C), the results are within the acceptance criteria and no significant change of the ADC profile is pointed out.

After **48 months** of storage and for all the tested batches (i.e. batch 2) in long term storage conditions (5°C ± 3°C), the results are within the acceptance criteria and no significant change of the ADC profile is pointed out.

A summary of stability data obtained with batch 2 is presented in Tables 15 and 16 below.

**Table 15: Summary of stability data at 5°C batch 2 for storage of 1 to 9 months**

| **Tests** | | **Acceptance criteria** | **T0** | **6 months** | **9 months** |
|---|---|---|---|---|---|
| **APPEARANCE** | | | | | |
| Content | | Clear to slightly opalescent, colourless to pale yellow solution | Clear and colourless solution | Slightly opalescent and colourless solution | Slightly opalescent and colourless solution |
| **IDENTITY of ADC** | | | | | |
| Fragment profile by LC | | Complies with reference | Complies | Complies | Complies |
| pH | | 6.0 to 7.0 | 6.5 | 6.5 | 6.5 |
| Average DAR | | 3.5 to 4.5 | 3.9 | 3.9 | 4.0 |
| Target binding - EC₅₀ ratio (in % of reference substance) by ELISA | | 50 to 150 | 100 | 79 | 107 |
| Biological activity (cytotoxicity) - IC50 ratio (in % of reference substance) | | 50 to 200 | 75 | 98 | 71 |
| Size variants (% area) (SEC-UV) | | | Method 1 | Method 1 | Method 1 |
| | Monomer | ≥ 90 | 98 | 98 | 98 |
| | Aggregates | ≤ 5 | 1 | 2 | 2 |
| **TESTS** | | | | | |
| Drug loading and distribution by HIC | | | | | |
| | D0 (%) | ≤ 10 | 5 | 6 | 6 |
| | D1 | | 1 | 2 | 2 |
| | D2 | | 28 | 28 | 27 |
| | D3 | | 3 | 3 | 3 |
| | D4 | | 35 | 34 | 33 |
| | D5 | | 0 | 0 | 0 |
| | D6 | | 19 | 17 | 18 |
| | D7 | | 0 | 0 | 0 |
| | D8 | | 9 | 10 | 12 |
| Residual drug and related substances by LC (Sum % w/w (drug/ADC)) | | ≤ 0.1 | < 0.1 | < 0.1 | < 0.1 |
| Particulate contamination : sub-visible particles (/vial) (Ph. Eur. 2.9.19 Method 2) | | | | | |
| | ≥ 10 µm | ≤ 3000 | 9 | 50 | NP |
| | ≥ 25 µm | ≤ 300 | 2 | 10 | |
| Bacterial endotoxins (IU/mg ADC) | | < 0.5 | < 0.5 | < 0.5 | NP |

| **Tests** | **Acceptance criteria** | **T0** | **6 months** | **9 months** | |
|---|---|---|---|---|---|
| Sterility | Sterile | Sterile | NP | NP | |
| **QUANTITY** | | | | | |
| Protein content by UV (mg/mL) | 4.5 to 5.5 | 5.3 | 5.1 | 5.1 | |

| | | | | | |
|---|---|---|---|---|---|
| NP = Not performed | | | | | |

**Table 16: Summary of stability data at 5°C batch 2 for storage of 12 to 48 months**

| **Tests** | | **Acceptance criteria** | **12 months** | **18 months** | **24 months** | **36 months** | **48 months** |
|---|---|---|---|---|---|---|---|
| **APPEARANCE** | | | | | | | |
| | Content | Clear to slightly opalescent, colourless to pale yellow solution | Clear and colourless solution | Slightly opalescent and colourless solution | Clear and colorless solution | Clear and colourless solution | Pale yellow and slightly opalescent solution |
| **IDENTITY of ADC** | | | | | | | |
| Fragment profile by LC | | Complies with reference | Complies | Complies | Complies | Complies | Complies |
| pH | | 6.0 to 7.0 | 6.5 | 6.4 | 6.6 | 6.4 | 6.5 |
| Average DAR | | 3.5 to 4.5 | 3.9 | 3.9 | 3.8 | 4.1 | 3.9 |
| Target binding - EC₅₀ ratio (in % of reference substance) by ELISA | | 50 to 150 | 85 | 106 | 99 | 95 | 74 |
| Biological activity (cytotoxicity) - IC50 ratio (in % of reference substance) | | 50 to 200 | 118 | 81 | 77 | 101 | 77 |
| Size variants (% area) (SEC-UV) | | | Method 1 | Method 1 | Method 1 | Method 2 | Method 2 |
| | Monomer | ≥ 90 | 98 | 95 | 97 | 98 | 99 |
| | Aggregates | ≤ 5 | 1 | 2 | 2 | 1 | 1 |
| **TESTS** | | | | | | | |
| Drug loading and distribution by HIC | | | | | | | |
| | D0 (%) | ≤ 10 | 6 | 6 | 6 | 5 | 6 |
| | D1 | | 2 | 2 | 2 | 3 | 3 |
| | D2 | | 27 | 28 | 28 | 25 | 27 |
| | D3 | | 4 | 4 | 3 | 2 | 4 |
| | D4 | | 34 | 33 | 34 | 33 | 33 |
| | D5 | | 0 | 0 | 0 | 0 | ND |
| | D6 | | 19 | 17 | 19 | 19 | 19 |
| | D7 | | 0 | 0 | 0 | 0 | ND |
| | D8 | | 9 | 11 | 8 | 13 | 10 |
| Residual drug and related substances by LC (Sum % w/w (drug/ADC)) | | ≤ 0.1 | < 0.1 | < 0.1 | < 0.1 | < 0.1 | < 0.1 |
| Particulate contamination: sub-visible particles (/vial) (Ph. Eur. 2.9.19 Method 2) | | | | | | | |
| | ≥ 10 µm | ≤ 3000 | 59 | NP | 7 | 5 | 6 |
| | ≥ 25 µm | ≤300 | 12 | | 1 | 3 | 3 |
| Bacterial endotoxins (IU/mg ADC) | | < 0.5 | < 0.5 | NP | < 0.5 | < 0.5 | < 0.5 |
| Sterility | | Sterile | Sterile | NP | Sterile | Sterile | Sterile |
| **QUANTITY** | | | | | | | |
| Protein content by UV (mg/mL) | | 4.5 to 5.5 | 5.0 | 5.1 | 5.2 | 5.1 | 5.1 |

| | | | | | | | |
|---|---|---|---|---|---|---|---|
| NP = Not performed | | | | | | | |

### Conclusion on Shelf-Life

Considering these stability data, the ADC drug product for solution for infusion has a shelf life of at least 48 months with the storage conditions at 5°C ± 3°C.

## Claims

1. Use of a liquid aqueous composition comprising a tonicity agent, a buffering agent, a non-ionic surfactant and a polysaccharide, and having a pH of from 5 to 7, for solubilization and stabilization of an antibody-drug conjugate during a period T equal or greater than 3 months at a temperature Θ of from 2°C to 8°C**.**

2. The use according to claim 1, wherein the tonicity agent is selected in the group consisting of polyols, inorganic salts, non aromatic amino acids, and mixtures thereof.

3. The use according to claim 1 or claim 2, wherein the tonicity agent is a polyol selected in the group consisting of glycerol, erythritol, arabitol, xylitol, sorbitol, mannitol, and mixtures thereof.

4. The use according to any one of claim 1 to claim 2, wherein the tonicity agent is an inorganic salt selected in the group consisting of sodium chloride, sodium sulphate, potassium chloride, potassium sulphate, magnesium chloride, magnesium sulphate, calcium chloride, calcium sulphate, and mixtures thereof, preferably is NaCl, MgCl₂ or CaCl₂.

5. The use according to claim 4, wherein the tonicity agent is NaCl at a concentration of from 100 mM to 200 mM, preferably at about 150 mM.

6. The use according to any one of claim 1 to claim 5, wherein the buffering agent is selected in the group consisting of citrate buffer, phosphate buffer, and histidine buffer, preferably is a histidine buffer.

7. The use according to claim 6, wherein the buffering agent is a histidine buffer at a concentration of from 5 mM to 50 mM, preferably at about 25 mM.

8. The use according to any one of claim 1 to claim 7, wherein the non-ionic surfactant is a polysorbate selected in the group consisting of Polysorbate 20 and Polysorbate 80, preferably is Polysorbate 80.

9. The use according to claim 8, wherein the non-ionic surfactant is Polysorbate 80 at a concentration of from 0.0001 to 0.01% (v/v), preferably at about 0,005% (v/v).

10. The use according to any one of claim 1 to claim 9, wherein the polysaccharide is a disaccharide, preferably is sucrose.

11. The use according to claim 10, wherein the polysaccharide is a sucrose at a concentration of from 0.1% to 10% (w/v), preferably at about 6% (w/v).

12. The use according to any one of claim 1 to claim 10, wherein the pH is from 6 to 7 and preferably is about 6.5.

13. The use according to any one of claim 1 to claim 12, wherein the liquid aqueous composition comprises 150 mM NaCl, 25 mM Histidine buffer, 0.005% Polysorbate 80 (v/v), and 6% sucrose(w/v), and wherein the pH is 6.5.

14. The use according to any one of claim 1 to claim 13, wherein the period T is equal or greater than 24 months, preferably equal or greater than 36 months, and preferably equal or greater than 48 months.

15. The use according to any one of claim 1 to claim 14, wherein the period T is from 12 to 48 months.

16. The use according to any one of claim 1 to claim 15, wherein the antibody-drug conjugate is at a concentration of from 1 mg/mL to 10 mg/mL, preferably from 3 mg/mL to 7 mg/mL and more preferably at about 5 mg/mL.

17. The use according to any one of claim 1 to claim 16, wherein the antibody-drug conjugate corresponds to the following formula: Ab-(L-D)n or a pharmaceutically acceptable salt thereof, wherein Ab is an antibody, or an antigen binding fragment thereof; L is a cleavable or non-cleavable linker; D is a drug moiety; and n is an integer from 1 to 12.

18. The use according to claim 17, wherein Ab is capable of binding to the human IGF-IR and is an antibody, or an antigen binding fragment thereof, comprising the three heavy chain CDRs of sequence SEQ ID No. 1, 2 and 3 and the three light chain CDRs of sequence SEQ ID No. 4, 5 and 6.

19. The use according to any one of claim 17 to claim 18, wherein the linker L is selected from: 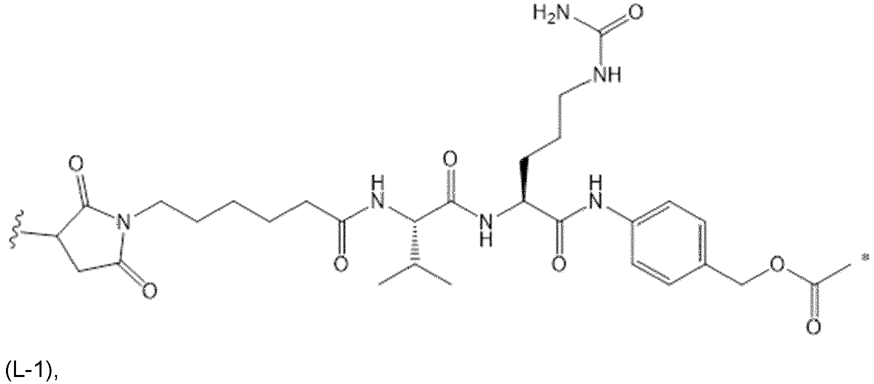 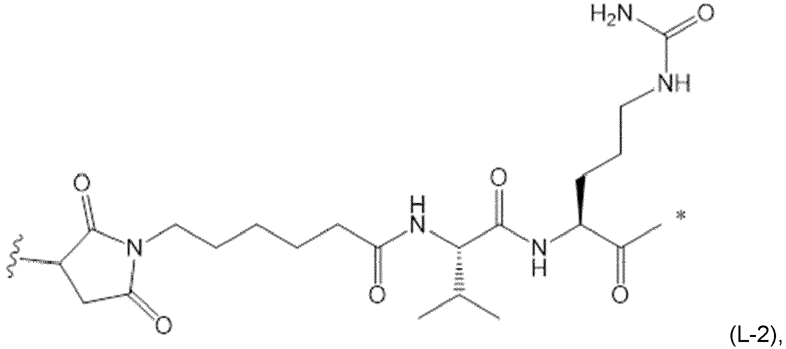 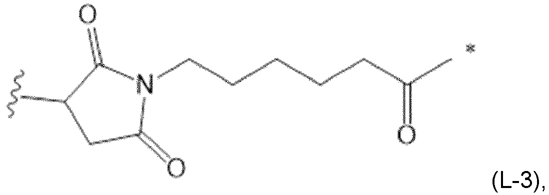 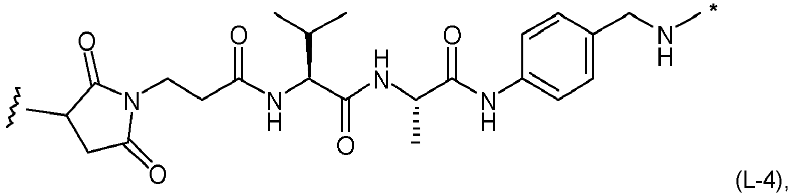 and 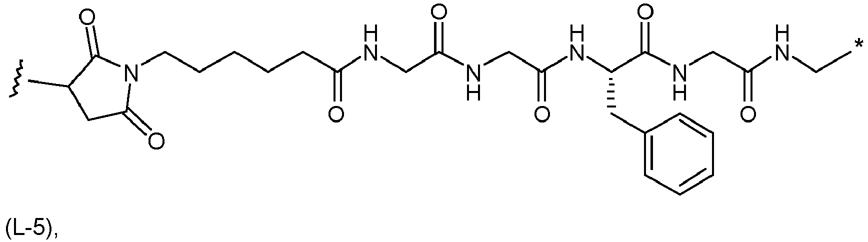 wherein the asterisk indicates the point of attachment to D, and the wavy line indicates the point of attachment to Ab.

20. The use according to any one of claim 17 to claim 19, wherein the drug D is a peptide-based drug, in particular selected from a dolastatin, an auristatin such as monomethylauristatine E or F, an amatoxin such as an amanitin, and derivatives thereof

21. The use according to any one of claim 17 to claim 20, wherein the antibody-drug conjugate comprises a DAR of from 1 to 8, preferably from 2 to 6 and more preferably of 4 ± 0.5.

22. The use according to any one of claim 1 to claim 21, wherein the stabilization of the antibody-drug conjugate is defined as:
- a percentage of aggregates of less than 5% of aggregates; and/or
- a percentage of monomers of at least 90% of monomers; and/or
- a percentage of free drug into the composition equal or less than 0.1% of antibody-drug conjugate.
